# EUROPEAN PATENT SPECIFICATION

(11) **EP 2 669 267 B1**
(45) Date of publication and mention of the grant of the patent: **10.01.2018**
(21) Application number: 13166056.5
(22) Date of filing: 12.11.2010
(51) Int. Cl.: C07C 215/10, A61K 31/133, A61P 35/00

(54) **Compositions and methods for treating hyperproliferative disorders**
Zusammensetzungen und Verfahren zur Behandlung hyperproliferativer Erkrankungen
Compositions et procédé de traitement de troubles hyperprolifératifs

(30) Priority: 12.11.2009 US 260595 P
(43) Date of publication of application: 04.12.2013
(62) Divisional of application: 10830833.9
(73) Proprietor: TEXAS TECH UNIVERSITY, Lubbock, TX 79409 (US)
(72) Inventor: Maurer, Barry, James, Idalou, TX 79329 (US); Reynolds, Charles, Patrick, Lubbock, TX 79416 (US)
(74) Representative: Lawrence, John

(56) References cited:
- WO-A1-00/00207
- WO-A1-01/47513
- DE JONGHE S ET AL: "Structure-Activity Relationship of Short-Chain Sphingoid Bases as Inhibitors of Sphingosine Kinase", BIOORGANIC & MEDICINAL CHEMISTRY LETTERS, PERGAMON, ELSEVIER SCIENCE, GB, vol. 9, no. 21, 1 November 1999 (1999-11-01), pages 3175-3180, XP004181029, ISSN: 0960-894X, DOI: 10.1016/S0960-894X(99)00554-5
- DATABASE CA [Online] CHEMICAL ABSTRACTS SERVICE, COLUMBUS, OHIO, US; DU, HONG-WANG ET AL: "Stereoselective synthesis of L-threo-dihydrosphingosines", XP002694401, retrieved from STN Database accession no. 1999:261609 & DU, HONG-WANG ET AL: "Stereoselective synthesis of L-threo-dihydrosphingosines", GAODENG XUEXIAO HUAXUE XUEBAO , 20(4), 590-592 CODEN: KTHPDM; ISSN: 0251-0790, 1999, XP002694402, -& DATABASE REGISTRY [Online] CHEMICAL ABSTRACTS SERVICE, COLUMBUS, OHIO, US; 26 June 1999 (1999-06-26), XP002694403, Database accession no. 226418-40-2 -& DATABASE REGISTRY [Online] CHEMICAL ABSTRACTS SERVICE, COLUMBUS, OHIO, US; 26 June 1999 (1999-06-26), XP002694404, Database accession no. 226418-37-7 -& DATABASE REGISTRY [Online] CHEMICAL ABSTRACTS SERVICE, COLUMBUS, OHIO, US; 16 August 1991 (1991-08-16), XP002694408, Database accession no. 135557-88-9
- MAJHOFER-ORESCANIN, B. ET AL: "Sphingolipide series. XVII. Synthesis and resolution of erythro- and threo-C20-dihydrosphingosines", TETRAHEDRON , 12, 56-62 CODEN: TETRAB; ISSN: 0040-4020, 1961, XP002694405,
- MORITA M ET AL: "Synthesis of alpha-, beta-monoglycosylceramides and four diastereomers of an alpha-galactosylceramide", BIOORGANIC & MEDICINAL CHEMISTRY LETTERS, PERGAMON, ELSEVIER SCIENCE, GB, vol. 5, no. 7, 6 April 1995 (1995-04-06), pages 699-704, XP004135577, ISSN: 0960-894X, DOI: 10.1016/0960-894X(95)00097-D
- B. J. MAURER ET AL: "Synergistic Cytotoxicity in Solid Tumor Cell Lines Between N-(4-Hydroxyphenyl)retinamide and Modulators of Ceramide Metabolism", JNCI JOURNAL OF THE NATIONAL CANCER INSTITUTE, vol. 92, no. 23, 6 December 2000 (2000-12-06), pages 1897-1909, XP055057535, ISSN: 0027-8874, DOI: 10.1093/jnci/92.23.1897

## Description

### FIELD OF THE INVENTION

The present invention relates to certain sphinganines and their use in chemotherapy regimens for the treatment of hyperproliferative disorders.

### BACKGROUND OF THE INVENTION

Sphinganines constitute a group of related long-chain aliphatic 2-amino-1,3-diols of which D-erythro-sphinganine (i.e, D-erythro-dihydrosphingosine = (2*S,*3*R)*-2-aminooctadecane-1,3-diol = D-erythro-2-amino-1,3-octadecanediol = (2S,3R)-2-amino-1,3-octadecanediol), an 18-carbon length sphinganine, is the most frequent naturally-occurring sphinganine in mammals. 20-carbon chain length D-erythro-sphinganine is also used in limited quantities in mammals and generally restricted to the central nervous system. Sphinganines are converted into dihydroceramides by acylation of their C2-amino group with fatty acids of varying chain length, generally 14 carbon to 30 carbon chain length. Dihydroceramides are further converted into ceramides by desaturation of the bond between Carbons 4 and 5 of the sphinganine backbone, i.e., the placement of a carbon-carbon double bond. Ceramides are predominantly used to make higher order sphingolipids, i.e. waxes, for the manufacture and repair of cellular membranes, and as signaling molecules. Historically, the term "ceramides" refers to both dihydroceramides and ceramides. The Carbon 4,5 double bound also distinguishes sphinganines from sphingosines. Historically, sphinganines and sphingosines are collectively referred to as "sphingoid bases". All naturally-occurring mammalian sphinganines and sphingosines are of D-erythro stereochemistry regarding the chirality of the C2-carbon amino group and C3-carbon hydroxyl group.

Safingol is the artificial, L-threo-stereochemical (diastereomer) variant of native, 18-carbon chain length, D-erythro-sphinganine. Safingol is also variously named L-threo-sphinganine = L*-threo* sphinganine (2S, 3S) = L-threo-dihydrosphingosine = L-threo-2-amino-1,3-octadecanediol = (2S ,3S)-2-amino-1,3-octadecanediol. Safingol has been reported to increase the anticancer activity of the retinoid (Vitamin A-derivative), fenretinide.

Fenretinide [HPR; all-*trans*-N-(4-hydroxyphenyl) retinamide; CAS Registry number 65646-68-6] is currently believed to effect cytotoxicity in cancer cells by generating reactive oxygen species and by increase of dihydroceramides. *See, e.g.,* D. Delia et al., Carcinogenesis 18, 943-948 (1997); N. Oridate et al., J. Natl. Cancer Inst. 89, 1191-1198

(1997).

U.S. Patent No. 4,665,098 to Gibbs describes pharmaceutical compositions of fenretinide as useful for the treatment of breast and bladder cancer.

U.S. Patent No. 7,169,819 to Gupta et al. describes pharmaceutical compositions of fenretinide suitable for the treatment of hyperproliferative disorders, including cancers.

U.S. Patent No. 5,821,072 to Schwartz et al. provides methods for screening protein kinase C inhibitors, including safingol, capable of potentiating apoptosis in tumor cells, along with methods for screening antitumor therapeutic agents suitable for combination therapy with a protein kinase C inhibitor capable of potentiating apoptosis in tumor cells.

U.S. Patent No. 6,352,844 to Maurer et al. provides for a method of treating hyperproliferative disorders, including cancers, by treating a patient in need of treatment with a ceramide-generating retinoid, such as fenretinide, with safingol.

De Jonghe, S et al., "Structure-Activity Relationship of Short-Chain Sphingoid Bases as Inhibitors of Sphingosine Kinase", Bioorganic & Medicinal Chemistry Letters, vol. 9, no. 21, pages 3175-3180 describes the synthesis of short-chain sphinganine analogues and investigations into their use as sphingosine kinase inhibitors in combination with sphingosine and fluorinated sphinganine analogues.

Du, Hong-Wang et al., "Stereoselective synthesis of L-threo-dihydrosphingosines", Gaodeng Xuexiao Huaxue Xuebao, 20(4), pages 590-592 describes a synthetic approach to L-*threo*-dihydrosphingosines from L-serine.

Majhofer-Orescanin, B. et al., "Sphingolipide series: XVII: Synthesis and resolution of erythro- and threo-C20-dihydrosphingosines", Tetrahedron, 12, pages 56-62 describes the synthesis and resolution of erythro- and threo-1,3-dihydroxy-2-amino-eicosane.

Morita, M et al., "Synthesis of alpha-, beta-monoglycosylceramides and four diastereomers of an alpha-galactosylceramide", Bioorganic & Medicinal Chemistry Letters, vol. 5, no. 7, pages 699-704 describes the synthesis of α-, β-galactosylceramides and α-, β-glucosylceramides, and four kinds of diastereomers of the ceramide portion in an α-galactosylceramide.

WO 00/00207 A1 and WO 01/47513 A1 describe methods of treating hyperproliferative disorders in a subject in need of such treatment, comprising administering to said subject, in combination, a treatment effective amount of: (a) a ceramide-generating retinoid such as fenretinide or a pharmaceutically acceptable salt thereof; and (b) at least one ceramide degredation inhibitor.

B. J. Maurer et al., "Synergistic Cytotoxicity in Solid Tumor Cell Lines Between N-(4-Hydroxyphenyl)retinamide and Modulators of Ceramide Metabolism", Journal of the National Cancer Institute, vol. 92, no. 23, pages 1897-1909 examines whether several molecules that inhibit enzymes involved in ceramide metabolism-L-*threo-*dihydrosphingosine, *d*,*l*-*threo*-1-phenyl-2-hexadecanoylamino-3-morpholino-1-propanol, and tamoxifen-enhanced 4-HPR-mediate cytotoxicity and/or affect ceramide levels.

### SUMMARY OF THE INVENTION

According to one aspect, the present invention provides a non-18 carbon chain length L-threo-sphinganine having the formula I: 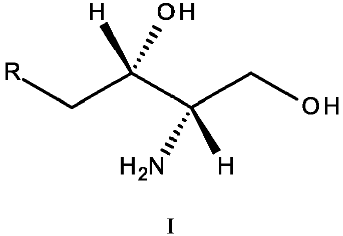 wherein R is a linear saturated hydrocarbon chain, and is such that the overall carbon chain length is 15 carbons, 17 carbons, 19 carbons, 20 carbons, or 21 carbons, for use in therapy.

According to another aspect, the invention provides a pharmaceutical L-threo-sphinganine composition comprising a ceramide-increasing retinoid and a non-18 carbon chain length L-threo-sphinganine derivative having the formula I, as defined above, in a pharmaceutical carrier.

According to a further aspect, the present invention provides a medicament for the use in a treatment of a hyperproliferative disorder in a subject, wherein the medicament comprises a ceramide-increasing retinoid and a non-18 carbon chain length L-threo-sphinganine having Formula I, as defined above, in a pharmaceutical carrier.

Examples of such L-threo-sphinganine compositions include L-threo-C20-sphinganine, also called, L-threo-icosasphinganine = L-threo-eicosasphinganine L-threo-2-amino-1,3-icosanediol = (2S,3S)-2-amino-1,3-icosanediol = L-threo-2-amino- 1,3-eicosediol = (2S,3S)-2-amino-1,3-eicosediol = (2S,3S)-2-amino-1,3-dihydroxy-eicosane = (2S,3S)-2-amino-1,3-dihydroxyeicosane;
L-threo-C19-sphinganine, also called, L-threo-2-amino-1,3-nonadecanediol = (2S,3S)-2-amino-1,3-nonadecanediol; and L-threo-C17-sphinganine, also called, L-threo-2- amino-1,3-heptadecanediol = (2S,3S)-2-amino-1,3-heptadecanediol.

The present invention is based on the unexpected discovery that such L-threo-sphinganines increase the anticancer properties of fenretinide in human cancer cell lines. Thus, the activity against hyperproliferative disorders, such as cancers, of fenretinide and other such retinoic acid derivatives that increase ceramides (i.e. dihydroceramides or ceramides) can be enhanced by administering in proximity such L-threo-sphinganines. Such administration can be sequentially, with the L-threo-sphinganine(s) administered prior to the ceramide-increasing retinoid, e.g. fenretinide, or concurrently, with the L-threo-sphinganine administered during part or all of the ceramide-increasing retinoid, e.g. fenretinide, administration period, or with the L-threo-sphinganine administered after the ceramide- increasing retinoid, e.g. fenretinide, so long as the beneficial effect is realized. Such L-threo-sphinganine(s) can be used in the treatment of hyperproliferative disorders wherein such L-threo- sphinganine(s) are administered with a ceramide-increasing retinoid, e.g. fenretinide, as described, together with an additional agent that manipulates cellular metabolism and cellular control of ceramide-generated cytotoxicity (e.g., a ceramide degradation inhibitor). Such agents as used in the claimed invention are (i) glucosylceramide and glucosyl(dihydro)ceramide synthase inhibitors and (ii) sphingomyelin and (dihydro)sphingomyelin synthase inhibitors. These may be administered alone or in combination with one another. Specific examples are given below. Preferably, the retinoic acid derivative that increases ceramides, e.g. fenretinide, is given in an amount effective to produce necrosis, apoptosis, autophagy, or other death-inducing process in the tumor cell, and the L-threo-sphinganine, with or without the ceramide degradation inhibitor, is given in an amount effective to increase the necrosis, apoptosis, authophagy or other death-inducing process in the tumor cell over that which would be produced by the retinoic acid derivative that increases ceramides, e.g. fenretinide, alone, or that expected to be produced by the sum of the retinoic acid derivative that increases ceramides, e.g. fenretinide, and the L-threo-sphinganine with or without the ceramide degradation inhibitor when given separately. The scope of the invention is defined by the claims, and provides medical uses of L-threo- sphinganines as defined in claim 1 and 3 and a pharmaceutical composition as defined in claim 2.

Thus the present invention includes compositions for use in treating a hyperproliferative disorder in a subject in need of such treatment, wherein the composition for administering to the subject, comprises, in combination, a treatment effective amount of: (a) a retinoic acid derivative that increases dihydroceramides or ceramides, such as fenretinide, or a pharmaceutically acceptable salt thereof; and (b) a non- 18 carbon chain length L-threo-sphinganine(s) of formula I as defined above or pharmaceutically acceptable salt thereof and, optionally, (c) a glucosylceramide or glucosyl(dihydro)ceramide synthesis inhibitor (including the pharmaceutically acceptable salts thereof), such as D-threo-1-phenyl-2- palmitoylamino-3-morpholino-1-propanol or a pharmaceutically acceptable salt thereof, and optionally, (d) a sphingomyelin or (dihydro)sphingomyelin synthase inhibitor. The synthesis inhibitor(s) is administered in an amount effective to enhance the activity of the retinoic acid derivative that increases ceramides and the L-threo-sphinganine, such that compounds together have an efficacious activity. Preferably, the retinoic acid derivative that increases ceramides and L-threo-sphinganine are given in an amount effective to produce necrosis, apoptosis, or autophagy other cell death process in the tumor cell, and the synthesis inhibitor is given in an amount effective to increase the necrosis, apoptosis, or autophagy or other cell death process produced in the tumor cell over that which would be expected by the retinoic acid derivative that increases ceramides and L-threo-sphinganine combined, or that expected to be produced by the sum of the retinoic acid derivative that increases ceramides and L-threo-sphinganine combination and the synthesis inhibitor when given separately. Other compounds, including the compounds described herein, may also be administered.

A theory of action is that the beneficial effect of L-threo-sphinganines on ceramide-increasing retinoids in human and canine hyperproliferative disorders is that such retinoids increase cellular D-erythro-dihydroceramides in susceptible hyperproliferative disorders, such as cancers, while L-threo-sphinganines are metabolically converted into L-threo-dihydroceramides to effect their beneficial actions. It is not concluded that the beneficial effect will be observed in all mammals, such as rodents, in which L-threo-sphinganines are metabolically converted into L-threo-ceramides. It is not excluded that L-threo-sphinganines perform a function(s) contributory to the function of the present invention that is distinct from its conversion into L-threo-dihydroceramides. Virtually all mammalian sphingo lipids are made using C-18 and C- 20 carbon length sphingoid backbones, and therefore it would not be expected that non-C 18 and C20 sphinganines, of any stereochemistry, would function at all. Further, C-20 backbone sphingolipids are not found outside the CNS in any appreciable quantity, and therefore it would not be expected that C-20-L-threo-sphinganines would function as is in fact observed as demonstrated herein.

The foregoing is illustrative of the present invention, and is not to be construed as limiting thereof. The invention is defined by the following claims.

### BRIEF DESCRIPTION OF THE DRAWINGS

For a more complete understanding of the present invention, reference is now made to the detailed description of the invention along with the accompanying figures in which:
**FIG. 1** is the dose-response of C17-L-threo-sphinganine in combination with fenretinide showing cytotoxicity at one or more doses in the drug-resistant CHLA-90 neuroblastoma cancer cell line.
**FIG. 2** is the dose-response of C17-L-threo-sphinganine in combination with fenretinide showing cytotoxicity at one or more doses in the CHLA-266 brain cancer (PNET) cell line in 2% oxygen.
**FIG. 3** is the dose-response of C17-L-threo-sphinganine in combination with fenretinide showing cytotoxicity at one or more doses in the GBM2 glioblastoma brain cancer cell line.
**FIG. 4** is the dose-response of C17-L-threo-sphinganine in combination with fenretinide showing cytotoxicity at one or more doses in the HT-29 colon cancer cell line in 2% oxygen.
**FIG. 5** is the dose-response of C17-L-threo-sphinganine in combination with fenretinide showing cytotoxicity at one or more doses in the COG-LL-317 Acute Lymphoblastic Leukemia (ALL) cancer cell line in 5% oxygen.
**FIG. 6** is the dose-response of C17-L-threo-sphinganine in combination with fenretinide showing cytotoxicity at one or more doses in the MOLT-4 ALL leukemia cell line.
**FIG. 7** is the dose-response of C19-L-threo-sphinganine in combination with fenretinide showing cytotoxicity at one or more doses in drug resistant the CHLA-90 neuroblastoma cancer cell line.
**FIG. 8** is the dose-response of C19-L-threo-sphinganine in combination with fenretinide showing cytotoxicity at one or more doses in the CHLA-266 brain cancer (PNET) cell line in 2% oxygen.
**FIG. 9** is the dose-response of C19-L-threo-sphinganine in combination with fenretinide showing cytotoxicity at one or more doses in the GBM2 glioblastoma brain cancer cell line.
**FIG. 10** is the dose-response of C19-L-threo-sphinganine in combination with fenretinide showing cytotoxicity at one or more doses in the HT-29 colon cancer cell line.
**FIG. 11** is the dose-response of C19-L-threo-sphinganine in combination with fenretinide showing cytotoxicity at one or more doses in the COG-LL-317 ALL leukemia cell line in 5% oxygen.
**FIG. 12** is the dose-response of C20-L-threo-sphinganine in combination with fenretinide showing cytotoxicity at one or more doses in the drug resistant CHLA-90 neuroblastoma cell line.
**FIG. 13** is the dose-response of C20-L-threo-sphinganine in combination with fenretinide showing cytotoxicity at one or more doses in the CHLA-266 brain cancer (PNET) cell line in 2% oxygen.
**FIG. 14** is the dose-response of C20-L-threo-sphinganine in combination with fenretinide showing cytotoxicity at one or more doses in the GBM2 glioblastoma brain cancer cell line.
**FIG. 15** is the dose-response of C20-L-threo-sphinganine in combination with fenretinide showing cytotoxicity at one or more doses in the HT-29 colon cancer cell line.
**FIG. 16** is the dose-response of C20-L-threo-sphinganine in combination with fenretinide showing cytotoxicity at one or more doses in the MCF-7/ADR (OVCAR-8/ADR) ovarian cancer cell line in 2% oxygen.
**FIG. 17** is the dose-response of C20-L-threo-sphinganine in combination with fenretinide showing cytotoxicity at one or more doses in the COG-LL-317 ALL cell line in 5% oxygen.
**FIG. 18** is the dose-response of C17-L-threo-sphinganines in combination with fenretinide and D-threo-PPMP showing synergistically increased cytotoxicity at most doses in the GBM2 brain cancer cell line in 2% oxygen.
**FIG. 19** is the dose-response of C19-L-threo-sphinganines in combination with fenretinide and D-threo-PPMP showing synergistically increased cytotoxicity at most doses in the GBM2 brain cancer cell line in 2% oxygen.
**FIG. 20** is the dose-response of C20-L-threo-sphinganines in combination with fenretinide and D-threo-PPMP showing synergistically increased cytotoxicity at most doses in the GBM2 brain cancer cell line in 2% oxygen.
**FIG. 21** is the dose-response of C17-L-threo-sphinganines in combination with fenretinide and D-threo-PPMP showing synergistically increased Cytotoxicity at most doses in the HT-29 colon cancer cell line in 20% oxygen.
**FIG. 22** is the dose-response of C19-L-threo-sphinganines in combination with fenretinide and D-threo-PPMP showing synergistically increased cytotoxicity at most doses in the HT-29 colon cancer cell line in 20% oxygen.
**FIG. 23** is the dose-response of C20-L-threo-sphinganines in combination with fenretinide and D-threo-PPMP showing synergistically increased cytotoxicity at most doses in the HT-29 colon cancer cell line in 2% oxygen.
**FIG. 24** is the dose-response of C17-L-threo-sphinganines in combination with fenretinide and D-threo-PPMP showing synergistically increased cytotoxicity at most doses in the MOLT-4 ALL leukemia cell line in 2% oxygen.
**FIG. 25** is the dose-response of C19-L-threo-sphinganines in combination with fenretinide and D-threo-PPMP showing synergistically increased cytotoxicity at most doses in the MOLT-4 ALL leukemia cell line in 2% oxygen.
**FIG. 26** is the dose-response of C20-L-threo-sphinganines in combination with fenretinide and D-threo-PPMP showing synergistically increased cytotoxicity at most doses in the MOLT-4 ALL leukemia cell line in 2% oxygen.

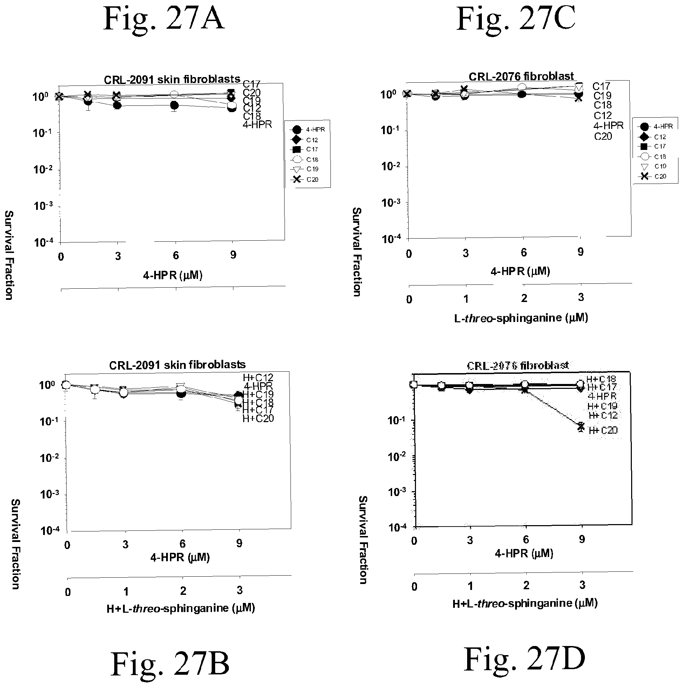

**FIGS. 27A - 27D** show the results of Fenretinide and L-*threo*-sphinganines tested in normal human fibroblast (normal skin cell) cell lines, CRL-2091 and CRL-2076.
**FIGS. 28A - 28D** show the results of Fenretinide and L-*threo*-sphinganines tested in human Multiple Myeloma (a cancer of the blood and bone marrow) cell line, RPMI-8226.
**FIGS. 29A - 29D** show the results of Fenretinide and L-*threo*-sphinganines tested in human Multiple Myeloma (a cancer of the blood and bone marrow) cell line, U-266.
**FIGS**. **30A** - **30D** show the results of Fenretinide and L-*threo*-sphinganines tested in human Glioblastoma multiforme (brain cancer) cell line, A-172.
**FIGS**. **31A - 31D** show the results of Fenretinide and L-*threo*-sphinganines tested in human Glioblastoma (brain cancer) cell line, U-118.

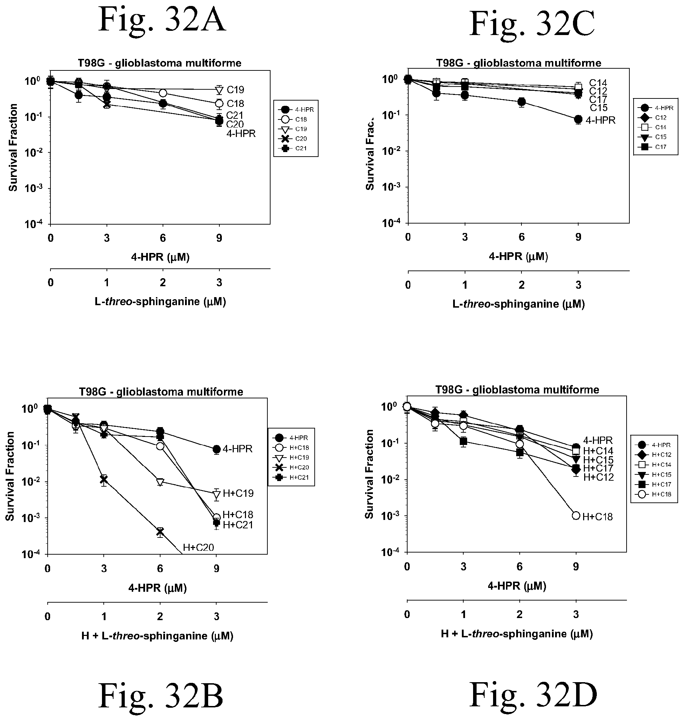

**FIGS. 32A - 32D** show the results of Fenretinide and L-*threo*-sphinganines tested in human Glioblastoma multiforme (brain cancer) cell line, T98G.

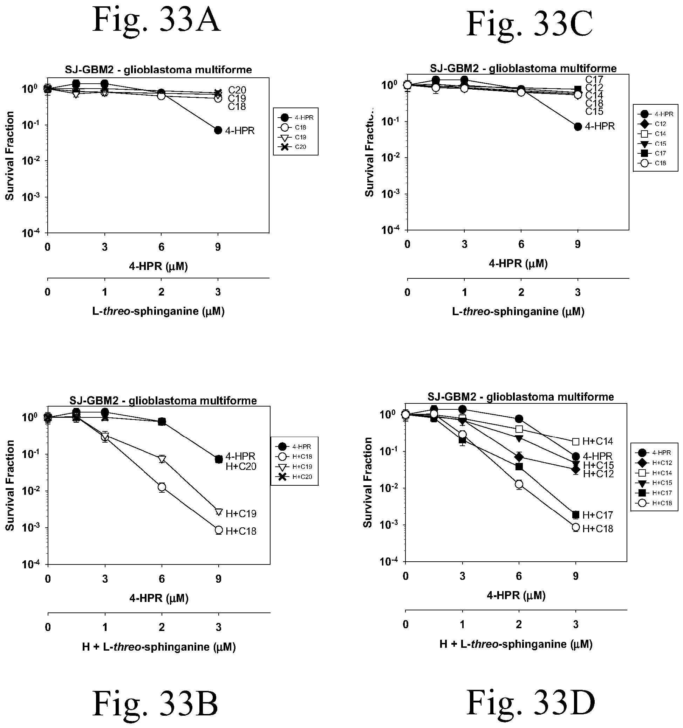

**FIGS. 33A - 33D** show the results of Fenretinide and L-*threo*-sphinganines tested in human Glioblastoma multiforme (brain cancer) cell line, SJ-GBM2.

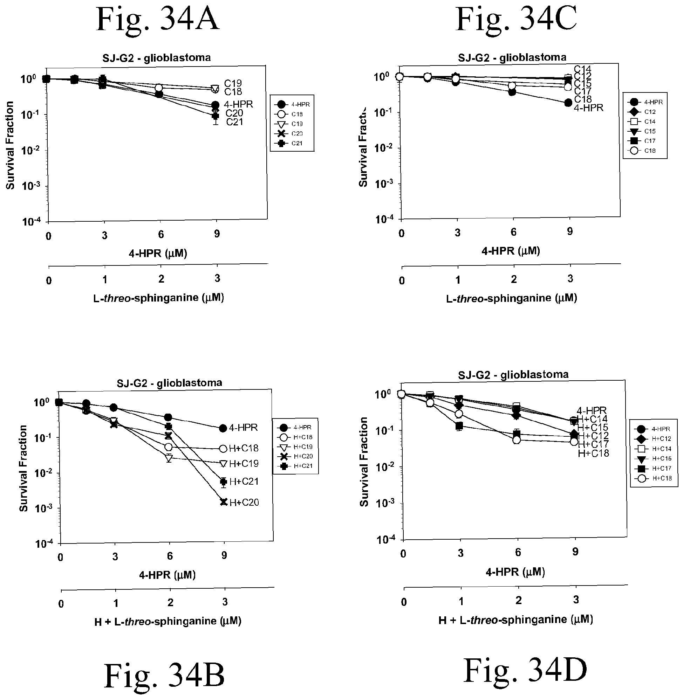

**FIGS. 34A - 34D** show the results of Fenretinide and L-*threo*-sphinganines tested in human Glioblastoma (brain cancer) cell line, SJ-G2.

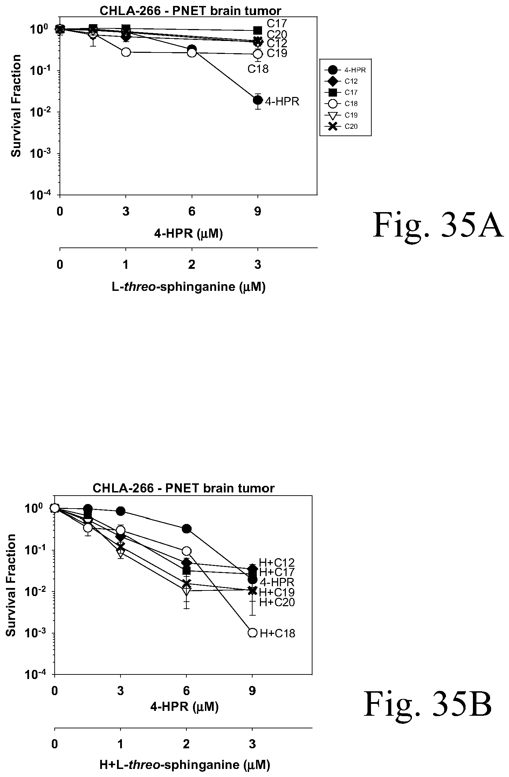

**FIGS. 35A** - **35B** show the results of Fenretinide and L-*threo*-sphinganines tested in human primitive neuroectodermal tumor (PNET) (brain cancer) cell line, CHLA-266.

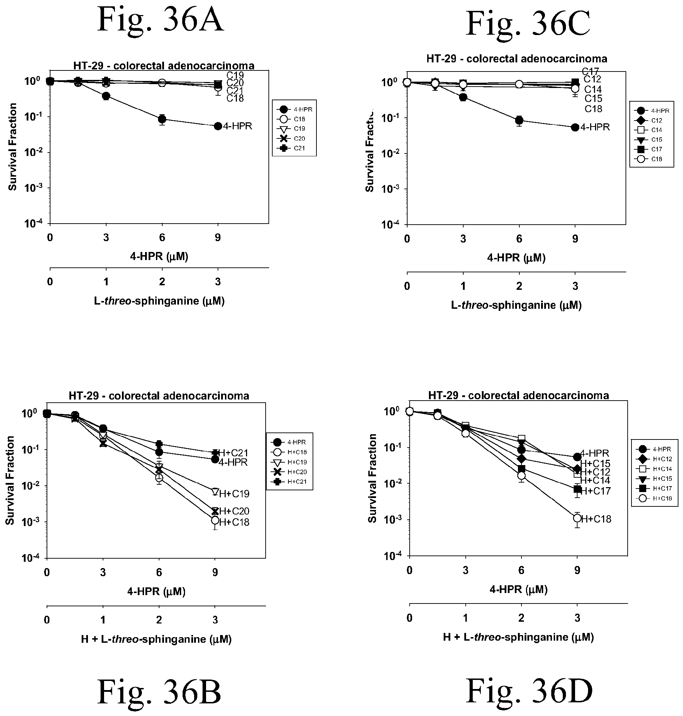

**FIGS. 36A - 36D** show the results of Fenretinide and L-*threo*-sphinganines tested in human colorectal adenocarcinoma (colon cancer) cell line, HT-29.

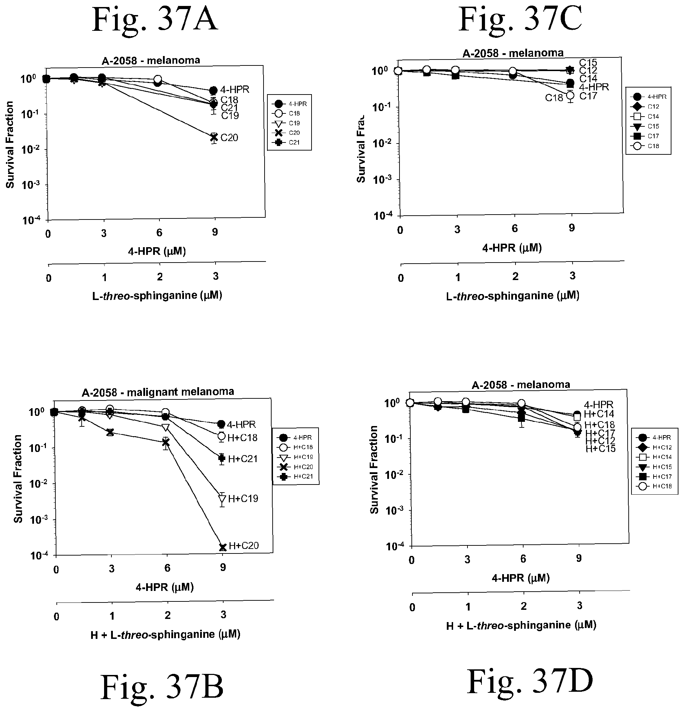

**FIGS. 37A - 37D** show the results of Fenretinide and L-*threo*-sphinganines tested in human melanoma (skin cancer) cell line, A-2058.

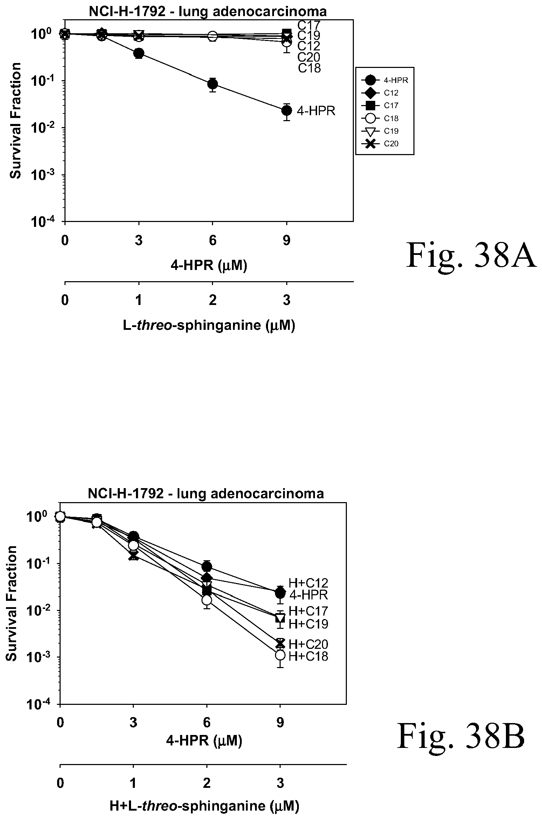

**FIGS. 38A - 38B** show the results of Fenretinide and L-*threo*-sphinganines tested in human lung adenocarcinoma (lung cancer) cell line, NCI-H-1792.

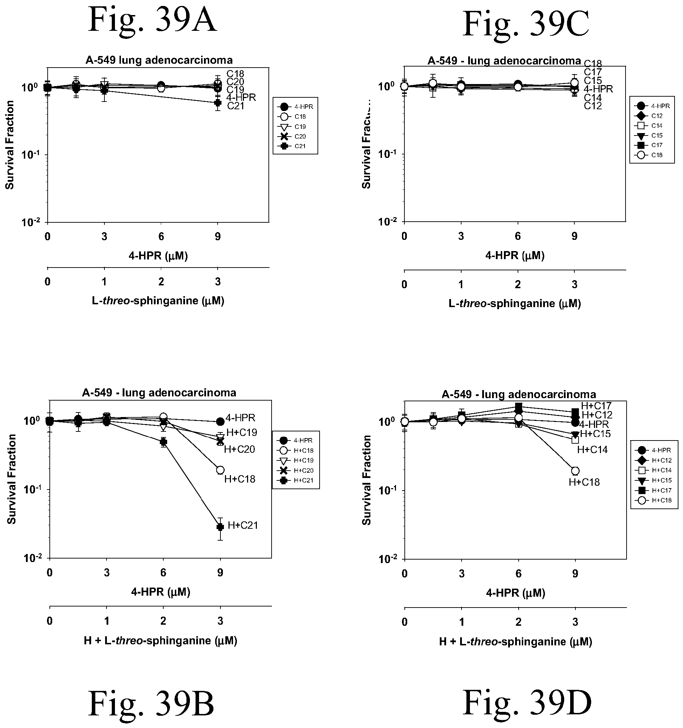

**FIGS. 39A - 39D** show the results of Fenretinide and L-*threo*-sphinganines tested in human lung adenocarcinoma (lung cancer) cell line, A-549.

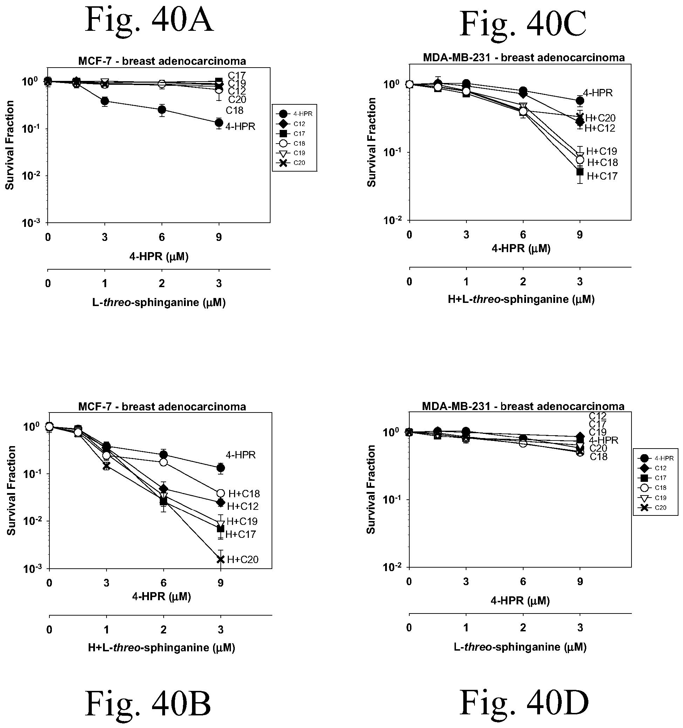

**FIGS. 40A - 40D** show the results of Fenretinide and L-*threo*-sphinganines tested in human breast adenocarcinoma (breast cancer) cell lines, MCF-7 and MDA-MB-231.

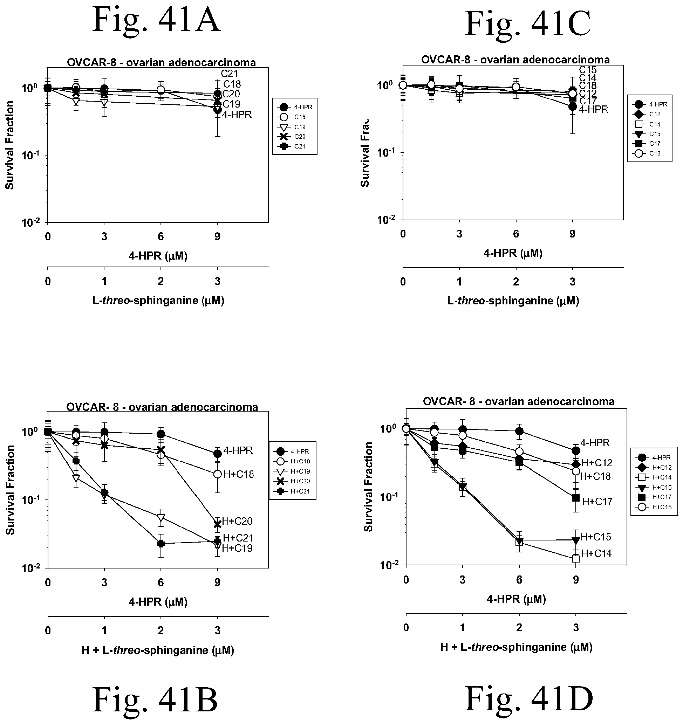

**FIGS. 41A - 41D** show the results of Fenretinide and L-*threo*-sphinganines tested in human ovarian adenocarcinoma (ovarian cancer) cell line, OVCAR-8.

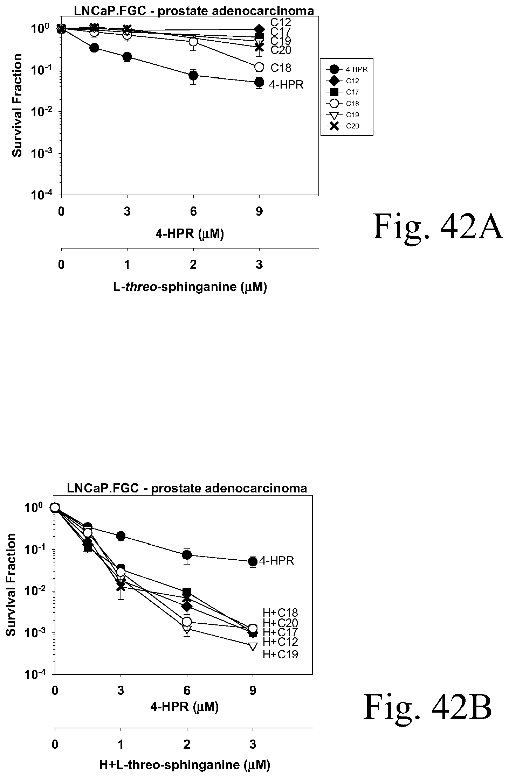

**FIGS. 42A - 42B** show the results of Fenretinide and L-*threo*-sphinganines tested in human prostate adenocarcinoma (prostate cancer) cell line, LNCaP.

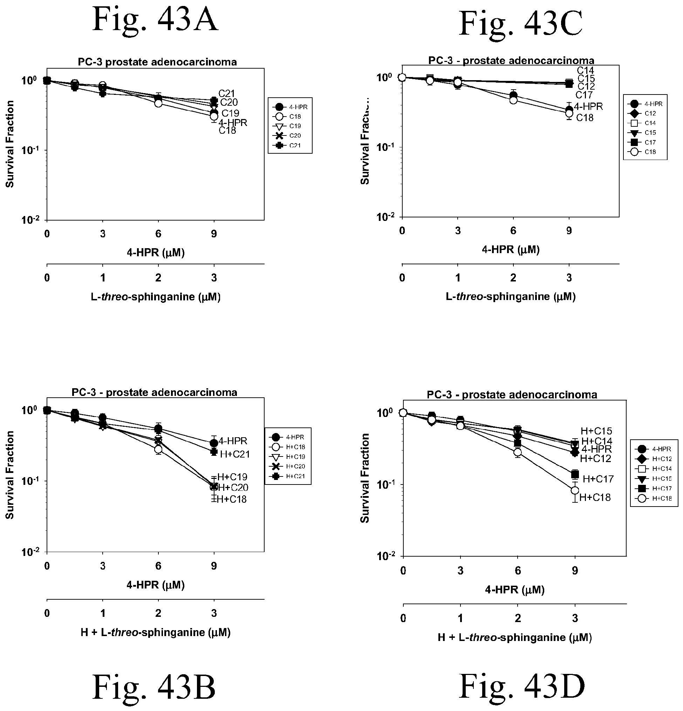

**FIGS. 43A - 43D** show the results of Fenretinide and L-*threo*-sphinganines tested in human prostate adenocarcinoma (prostate cancer) cell line, PC-3.

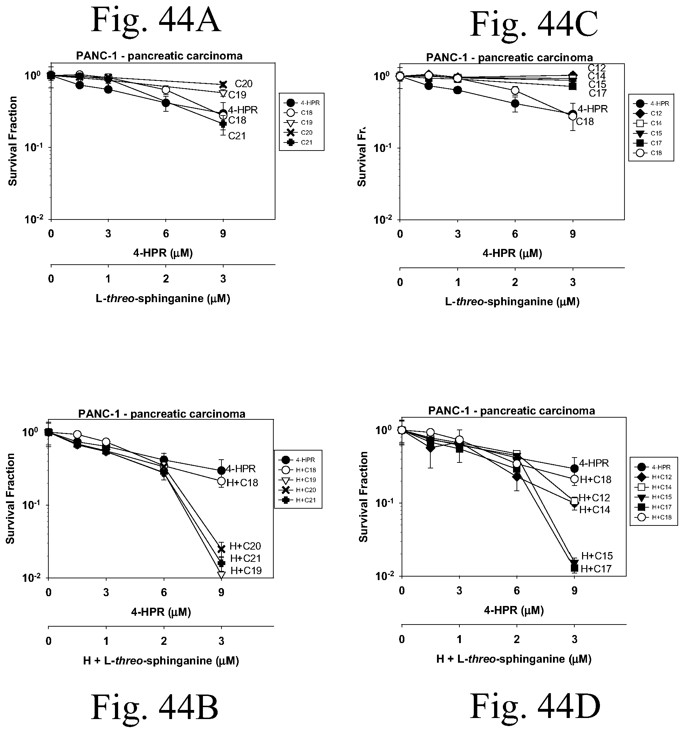

**FIGS. 44A - 44D** show the results of Fenretinide and L-*threo*-sphinganines tested in human pancreatic adenocarcinoma (pancreas cancer) cell line, PANC-1.

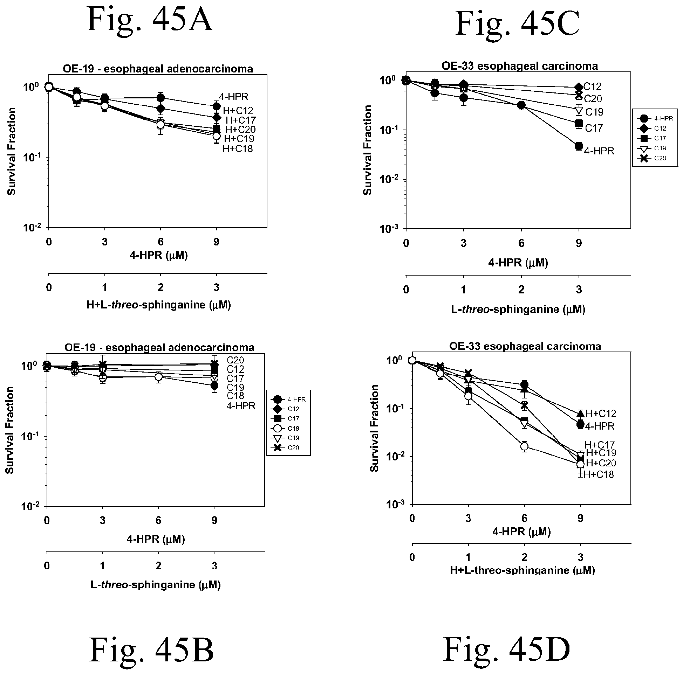

**FIGS. 45A - 45D** show the results of Fenretinide and L-*threo*-sphinganines tested in human esophageal adenocarcinoma (esophagus cancer) cell lines, OE-19 and OE-33.

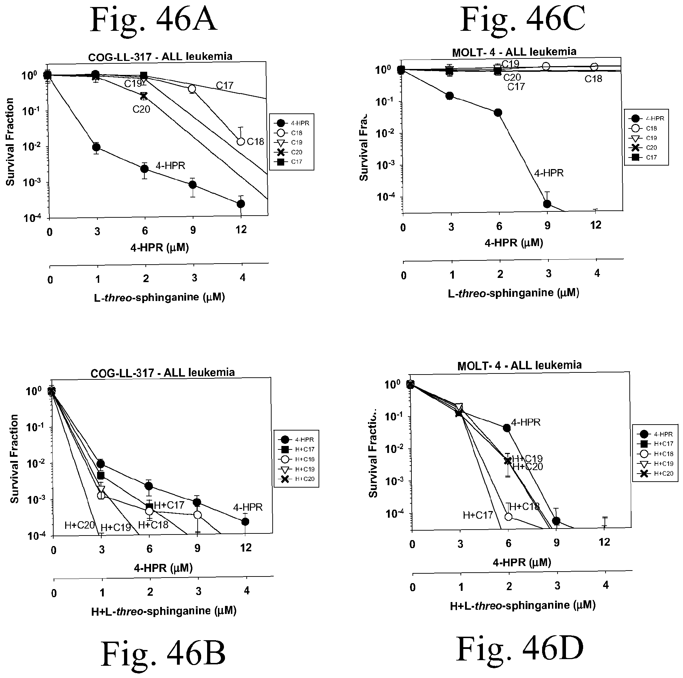

**FIGS. 46A - 46D** show the results of Fenretinide and L-*threo*-sphinganines tested in human acute lymphoblastic leukemia (pediatric ALL, a blood cancer) cell lines, COG-LL-317 and MOLT-4.

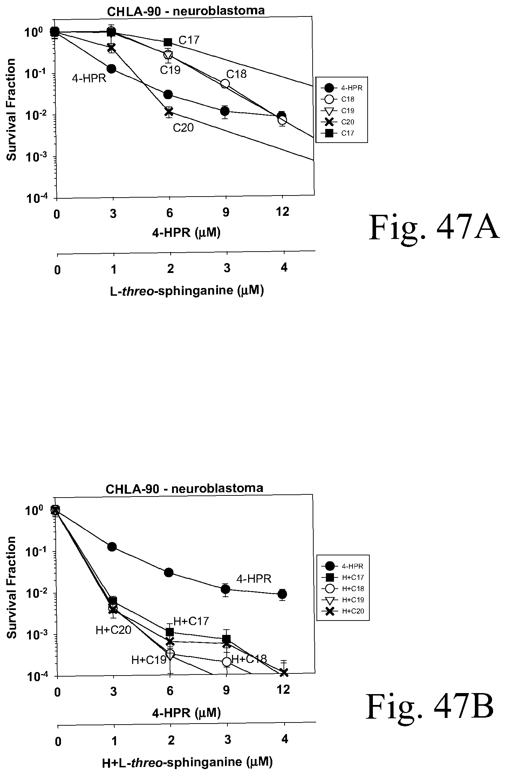

**FIGS. 47A - 47B** show the results of Fenretinide and L-*threo*-sphinganines tested in human pediatric neuroblastoma (a nerve-related, solid tumor cancer) cell line, CHLA-90.

### DETAILED DESCRIPTION OF THE INVENTION

Disclosed herein are certain sphinganines that can be used in combination chemotherapy regimens for the treatment of hyperproliferative disorders. The numerous innovative teachings of the present invention will be described with particular reference to several embodiments (by way of example, and not of limitation).

The disclosed combination of sphinganines and their use in chemotherapy regimens for the treatment of hyperproliferative disorders is generally described, with examples incorporated particular embodiments of the invention and to demonstrate the practice and advantages thereof. It is understood that the examples are given by way of illustration and are not intended to limit the specification or the claims in any manner. The scope of the invention is defined by the claims. No claim is made to a method of medical treatment. Any references to methods or treatments refer to the medical use of the compounds, pharmaceutical compositions and medicaments of the present invention.

The methods of the present invention utilize the combined effects of retinoic acid derivatives, such as fenretinide, coupled with the novel sphingoid bases described herein as potentiating agents, to manipulate cellular metabolism and cellular control of ceramide-generated toxicity, in order to inhibit or prevent the growth of tumors, cancers, neoplastic tissue and other premalignant and noneoplastic hyperproliferative disorders, all of which are together referred to as hyperproliferative or hyperplastic disorders herein. The treatments employed herein may be used to inhibit growth and/or to induce cytotoxicity (by necrotic or apoptotic mechanisms, or both) in the target cells, which are generally hyperproliferative cells (including tumors, cancers, and neoplastic tissue, along with pre-malignant and non-neoplastic or non-malignant hyperproliferative disorders).

Examples of tumors, cancers, and neoplastic tissue that can be treated by the present invention include malignant disorders such as breast cancers; osteosarcomas; angiosarcomas; fibrosarcomas and other sarcomas; leukemias; lymphomas; sinus tumors; ovarian, cervical, ureteral, bladder, prostate and other genitourinary cancers; colon, esophageal and stomach cancers and other gastrointestinal cancers; lung cancers; myelomas; pancreatic cancers; liver cancers; kidney cancers; endocrine cancers; skin cancers; and brain or central and peripheral nervous (CNS) system tumors, malignant or benign, including gliomas and neuroblastomas.

Examples of premalignant and non-neoplastic or non-malignant hyperproliferative disorders include myelodysplastic disorders; cervical carcinoma-in-situ; familial intestinal polyposes such as Gardner syndrome; oral leukoplakias; histiocytoses; keloids; hemangiomas; hyperproliferative arterial stenosis, inflammatory arthritis; hyperkeratoses and papulosquamous eruptions including arthritis. Also included are viral induced hyperproliferative diseases such as warts and EBV-induced disease (i.e., infectious mononucleosis) and scar formation. The compounds for use, pharmaceutical compositions and medicaments for use disclosed herein may be employed to treat any subject known or suspected of carrying or at risk of developing a hyperproliferative disorder as defined herein.

As used herein, "treatment" of a hyperproliferative disorder refers to methods of killing, inhibiting or slowing the growth or increase in size of a body or population of hyperproliferative cells or tumor or cancerous growth, reducing hyperproliferative cell numbers, or preventing spread to other anatomic sites, as well as reducing the size of a hyperproliferative growth or numbers of hyperpproliferative cells. As used herein, "treatment" is not necessarily meant to imply cure or complete abolition of hyperproliferative growths. As used herein, a treatment effective amount is an amount effective to result in the killing, the slowing of the rate of growth of hyperproliferative cells, the decrease in size of a body of hyperproliferative cells, and/or the reduction in number of hyperproliferative cells. The potentiating agent (or agents) is included in an amount sufficient to enhance the activity of the first compound, such that the two (or more) compounds together have greater therapeutic efficacy than the individual compounds given alone (e.g., due to synergistic interaction; reduced combined toxicity, etc.).

As used herein, the administration of two or more compounds "in combination" means that the two compounds are administered closely enough in time that the presence of one alters the biological effects of the other. The two compounds may be administered simultaneously (concurrently) or sequentially. Simultaneous administration may be carried out by mixing the compounds prior to administration, or by administering the compounds at the same point in time but at different anatomic sites or using different routes of administration.

The phrases "concurrent administration", "administration in combination", "simultaneous administration" or "administered simultaneously" as used herein, means that the compounds are administered at the same point in time or immediately following one another. In the latter case, the two compounds are administered at times sufficiently close that the results observed are indistinguishable from those achieved when the compounds are administered at the same point in time.

Subjects to be treated include both human subjects and animal subjects for veterinary purposes. Animal subjects are preferably mammalian subjects including horses, cows, dogs, cats, rabbits and sheep.

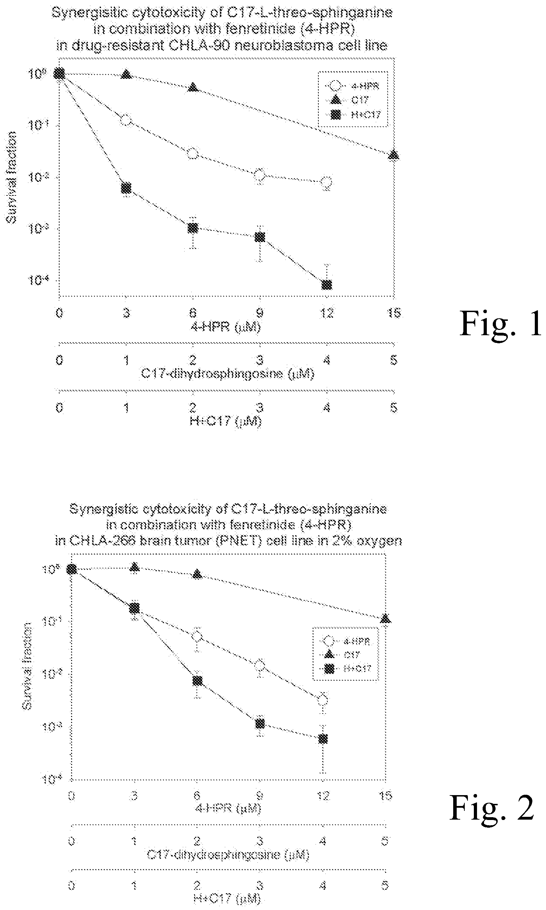

A variety of intracellular molecules are known to trigger or inhibit cell death (S. Rowan and D. Fisher, Leukemia 11, 457 (1997); K. Saini and N. Walker, Mol. Cell Biochem. 178, 9 (1998)). Most current work focuses on elucidating pathways for programmed cell death (apoptosis), in which triggers of apoptosis (such as DNA damage) can activate various pathways (e.g. p53, Fas, and others), which can be modulated by yet other molecules (such as the Bcl-2 family of pro-and anti-apoptotic proteins), with caspase activation being a late step in the final events leading to apoptotic cell death. However, not all cell death occurs via apoptosis, and cell death induced by 4-HPR involves both apoptosis and necrosis (J. Clifford et al., Cancer Res. 59, 14 (1999)) or autophagy (Zheng, W., et al., Biochim Biophys Acta. 1758:1864-84 (2006), Tiwari, M., et al., Carcinogenesis 29:600-9, (2008), Fazi, B., et al., Autophagy.4:435-41, (2008). The intracellular lipid ceramide is known to mediate apoptosis (L. Obeid et al., Science 259, 1769 (1993)(FIG. 1) and necrosis (Guo et al., Am. J. Physiol. 276, F390 (1999); Condorelli et al., Br. J. Pharmacol. 137, 75 (1999)). It has been shown to cause the apoptosis-inducing permeability transition of mitochondrial membranes (S. Susin et al., J. Exp. Med. 186, 25 (1997)), cause apoptosis-inducing ROS generation by mitochondrial complex III inhibition (A. Quillet-Mary et al., J. Biol. Chem. 272, 21388 (1997) and activate the pro-death JNK/SAPK pathway (S. Basu et al., Oncogene 17, 3277 (1998); T. Okazaki et al., Cell. Signal. 10, 685 (1998); W. Jarvis, Curr. Opin. Oncol. 10, 552 (1998)). Ceramide also activates a protein kinase (CAPK) (S. Mathias et al., Biochem. J. 335(Pt 3), 465 (1998) and a phosphorylase (PP2A) (L. Leoni et al., Biochem. Pharmacol. 55, 1105 (1998)) and can lead to the activation of the nuclear transcription factor, NF-kappaB (L. Johns et al., J. Immunol. 152, 5877 (1998); C. Gamard et al., J. Biol. Chem. 272, 1682 (1997)). Mechanisms by which cancer cells avoid the cytotoxic effects of ceramide can include metabolism to other forms, including nontoxic glucosylceramide (Y. Lavie et al., J. Biol. Chem. 272, 1682 (1997); Y. Lavie et al., J. Biol. Chem. 271, 19530 (1996); L. Yong-Yu et al., J. Biol. Chem. 274, 1140 (1999)) and sphingosine-1-phosphate. Sphingosine-1-phosphate opposes ceramide-induced cell death by activating the pro-life ERK1/2 pathway (O. Cuvillieret al., Nature 381, 800 (1996); O. Cuvillieret al., J. Biol. Chem. 273, 2910 (1998)). Thus, modulation of ceramide metabolism offers a means for enhancing the cytotoxic efficacy of 4-HPR (fenretinide) and other ceramide-generating retinoids.

Ceramide is generated intracellularly via activation of ceramide synthase, the de novo synthetic pathway or by activation of the neutral- or acidic- sphingomyelinases, leading to breakdown of sphingomyelin. Ceramide is metabolized to non-cytotoxic glucosylceramide by glucosylceramide synthase; and converted into cytotoxic sphingosine by alkaline- or acidic-ceramidases. Sphingosine is further converted to the anti-apoptotic sphingosine-1-phosphate by sphingosine kinase. We show below that modulation of these pathways can enhance, even synergistically enhance, the cytotoxicity of ceramide-generating retinoids such as 4-HPR (fenretinide).

Compounds that may be used to carry out the present invention, and formulations thereof and the manner of administering the same, are described in detail below.

### 1. Ceramide-generating Retinoids.

Ceramide-generating retinoids or retinoic acid derivatives that can be used to carry out the present invention are those generating ceramide in a host cell to which they are administered and include those described in U.S. Pat. No. 4,190,594 to Gander.

Ceramide-generating retinoids include all trans-retinoic acid (ATRA) and retinoic acid derivatives, including:
(A) esters of all-trans-retinoic acid having the following Formula II: 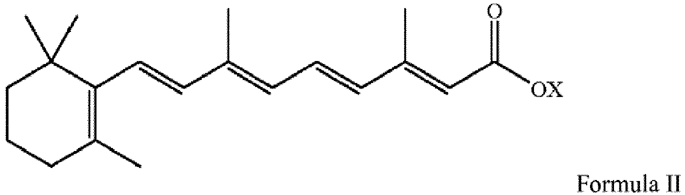 wherein X is a member selected from the group consisting of (Formulas III & IV): 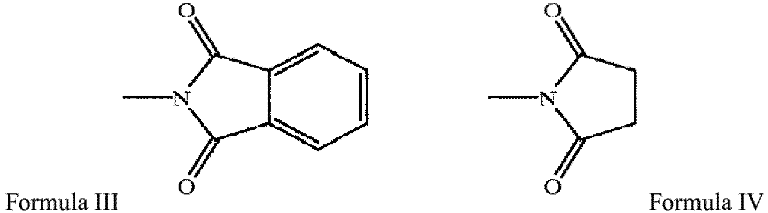
   2-cyclohexylethyl; 10-carbomethoxydecyl; 4-hydroxybutyl; cholesteryl; mixed m- and p-vinylbenzyl; and 4-bromobenzyl;
(B) esters of all-trans-retinoic acid having the following Formula V: 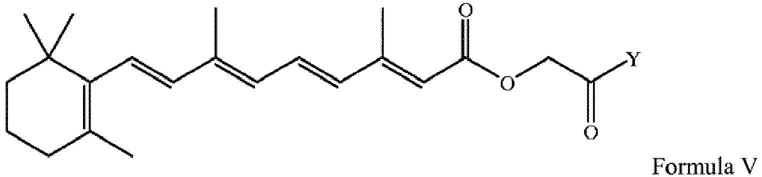 wherein Y is a member selected from the group consisting of: cholesteryloxy; phenyl; 4-bromophenyl; 4-methoxyphenyl; 4-nitrophenyl; 4-hydroxyphenyl; 4-methylphenyl; 4-cyanophenyl; 4-ethoxyphenyl; 4-acetoxyphenyl; 2-naphthyl; 4-biphenyl; 2,5-dimethoxyphenyl; 2,4-dichlorophenyl; 2,4-dimethylphenyl; 3,4-diacetoxyphenyl; 3,4,5-trimethoxyphenyl; and 2,4,6-trimethylphenyl; and
(C) amides of all-trans-retinoic acid having the following Formula VI: 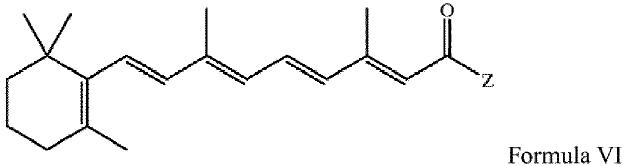 wherein Z is a member selected from the group consisting of: n-propylamino; tert-butylamino;1,1,3,3-tetramethylbutylamino; 1-morpholino; 4-hydroxyphenylamino; 4-carbomethoxy-2-hydroxyphenylamino; beta-(3,4-dimethoxyphenyl)-ethylamino; 2-benzothiazolylamino; 1-imidazolyl; 1-(2-nicotinoylhydrazolyl); 1-benzotriazolyl; 1-(1,2,4-triazolyl) (Formulas VII, VIII & IX);

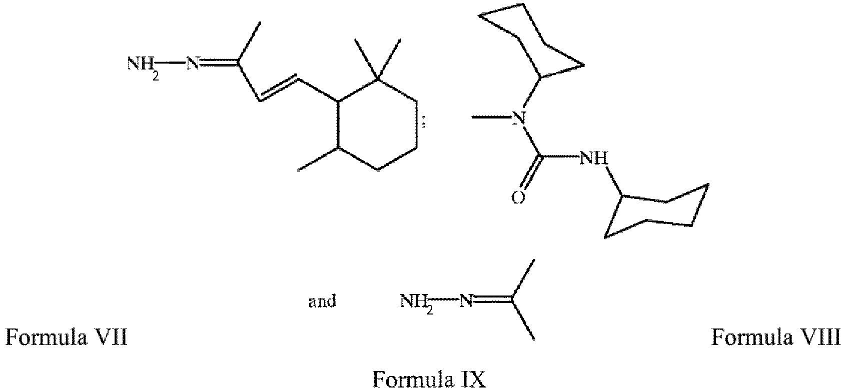

Particularly preferred is all-trans-N-(4-hydroxyphenyl)retinamide, also called fenretinide, which has CAS registry number 65646-68-6, and has the structure (Formula X):

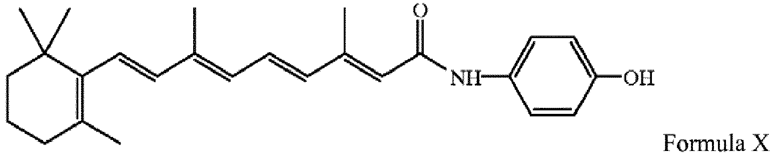

The foregoing compounds can be prepared in accordance with known techniques. See, e.g., U.S. Pat. No. 4,190,594 to Gander et al.; U.S. Pat. No. 4,665,098 to Gibbs.

Additional retinoic acid derivatives that can be used to carry out the present invention include C-Glycoside analogs of N-(4-hydroxyphenyl)retinamide-O-glucuronide. Such compounds and their preparation are known and described in U.S. Pat. Nos. 5,663,377 and 5,599,953, both to Curley et al.

Such compounds may have the general formula (Formula XI): 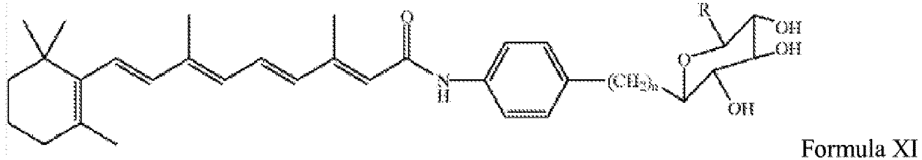 where R is COOH, CH.sub.2 OH, or H, and n is 0 or 1.

Specific examples of such compounds include: 4-(retinamido)phenyl-C-glucuronide; 4-(retinamido)phenyl-C-glucoside; 4-(retinamido)phenyl-C-xyloside; 4-(retinamido)benzyl-C-glucuronide; 4-(retinamido)benzyl-C-glucoside; 4-(retinamido)benzyl-C-xyloside; 1-(.beta.-D-glucopyranosyl) retinamide; and 1-(D-glucopyranosyluromosyl) retinamide.

### 2. Glucosylceramide Synthesis Inhibitors.

Any compound that inhibits glycosylceramide or glycosyl(dihydro)ceramide synthesis can be used, particularly glucosylceramide synthase inhibitors. Examples of such compounds include compounds having the formula (Formula XII): 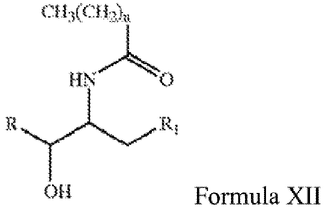 where R is an aromatic ring such as phenyl, a cyclohexyl group, or an alpiphatic group having 10 to 15 carbon atoms, R.sub.1 is an amine group such as a morpholino group; and n is an integer of from 4 to 18 (including functional homologues, isomers and pharmaceutically acceptable salts thereof. Preferably, n is 4, 6, 8, 10, 12 or 14, and the D enantiomer of such compounds are preferred. Such compounds are known and are disclosed, for example, in U.S. Pat. No. 5,302,609 to Shayman and Radin; U.S. Pat. No. 5,041,441 to Radin et al.; and U.S. Pat. No. 5,707,649 to Inokuchi et al. Specific examples of glucosylceramide synthase inhibitors include: 1-phenyl-2-acylamino-3-morpholino-1-propanol in which n is 6 to 12; 1-phenyl-2-decanoylamino-3-morpholino-1-propanol (PDMP); and 1-phenyl-2-palmitoylamino-3-morpholino-1-propanol (PPMP);

### 3. Additional Active Compounds and Screening.

Additional active compounds can be generated by known techniques, including rational drug design techniques and/or random drug design techniques (or combinatorial chemistry techniques).

In active compounds that interact with a receptor, the interaction takes place at the surface-accessible sites in a stable three-dimensional molecule. By arranging the critical binding site residues in an appropriate conformation, compounds which mimic the essential surface features of the active compound binding region may be designed and synthesized in accordance with known techniques. A molecule which has a surface region with essentially the same molecular topology to the binding surface of the active compound will be able to mimic the interaction of the active compound with its corresponding receptor. Methods for determining the three-dimensional structure of active compounds and producing active analogs thereof are known, and are referred to as rational drug design techniques. See, e.g., U.S. Pat. No. 5,593,853 to Chen; U.S. Pat. Nos. 5,612,895 and 5,331,573 to Balaji et al.; U.S. Pat. No. 4,833,092 to Geysen; U.S. Pat. No. 4,859,765 to Nestor; U.S. Pat. No. 4,853,871 to Pantoliano; and U.S. Pat. No. 4,863,857 to Blalock

In combinatorial chemistry (or random drug design) techniques, large combinatorial libraries of candidate compounds are screened for active compounds therein. Libraries used to carry out the present invention may be produced by any of a variety of split synthesis methods. Split synthesis methods in which a releasable tag is attached to the particle along with the organic compounds of interest are also known as cosynthesis methods. A variety of such methods are known. See, e.g., A. Furka et al., J. Pept. Protein Res. 37, 487 (1991); K. Lam et al., Nature 354, 82 (1991); R. Zuckermann et al., Int. J. pept. Protein Res. 40, 498 (1992); F. Sebestyen et al., Bioorg. Med. Chem. Lett. 3, 413 (1993); K. Lam et al., Bioorg. Med. Chem. Lett. 3, 419 (1993). For example, the library may be a library of organometallic compounds wherein the compound is a metal-ligand complex. The metal in the complex may be an early or late transition metal in high, low or zero oxidation states. The metal may also be any of the main group metals, alkali metals, alkaline earths, lanthanides or actinides. The ligand in the metal-ligand complex may be composed of, or derived from, chiral or achiral forms of cyclopentadienes, amino esters, oxazolidoinones, hydroxy acids, hydroxy esters, hydroxy amides, pyridines, fused pyridines, nitrogen heterocycles, oxazoles, imidazoles, pyrroles, crown ethers, cryptands, carcerands, phosphines, diphosphines, polyphosphines, quinuclidines, quinines, alkaloids, dextrins, cyclodextrins, salens, porpyrins, biaryls, sulfonamides, Schiff bases, metallocenes, monools, diols, polyols, amines, diamines, polyamines, ammonium salts, peptides, proteins and nucleic acids.

As a second example, the library may be a library of non-metal compounds including chiral or achiral forms of cyclopentadienes, amino esters, oxazolidinones, hydroxy acids, hydroxy esters, hydroxy amides, pyridines, fused pyridines, nitrogen heterocycles, oxazoles, imidazoles, pyrroles, crown ethers, cryptands, carcerands, phosphines, diphosphines, polyphosphines, quinuclidines, quinines, alkaloids, dextrins, cyclodextrins, salens, porphyrins, biaryls, sulfonamides, Schiff bases, metallocenes, monools, diols, polyols, amines, diamines, polyamines, ammonium salts, peptides, proteins and nucleic acids.

The solid supports may be separate from one another, or may be discreet regions on a surface portion of a unitary substrate, which surface portion may be positioned at the interface so that a plurality of the discreet regions are positioned at the interface. Such "chip-type" or "pin-type" solid supports are known. See, e.g., U.S. Pat. No. 5,288,514 to Ellman (pin-based support); U.S. Pat. No. 5,510,270 to Fodor et al. (chip-based support). Separate discreet supports (e.g., particles or beads) are currently preferred. Synthesis of the catalyst library and linking thereof to the discreet solid support may be carried out in accordance with known techniques, such as described in U.S. Pat. No. 5,565,324.

Compounds selected by any means, including those described above, may be screened for activity in increasing, including additively and synergistically increasing but preferably synergistically increasing, the cytostatic or cytotoxic activity of a ceramide-generating retinoid in tumor cells (or other hyperproliferative cells), by a method comprising: (a) contacting first control tumor cells with an amount of ceramide-generating retinoid (e.g., an amount that may or may not itself be effective to inhibit growth of said tumor cells); (b) contacting second control tumor cells with an amount of a test compound (eg., an amount that may or may not itself be effective to inhibit growth of said tumor cells); and (c) contacting experimental tumor cells with both said amount of ceramide generating retinoid in step (a) above and said amount of a test compound in step (b) above; and (d) determining the growth inhibition of said tumor cells of steps (a), (b) and (c) above; and then (e) comparing the growth inhibition or cytotoxic activity in the experimental tumor cells of step (c) with the growth inhibition of the control tumor cells of steps (a) and (b), a greater degree of growth inhibition determined in the experimental tumor cells of step (c) than the combined growth inhibition of the control tumor cells of steps (b) and (c) indicating that the test compound enhances the activity of the ceramide-generating retinoid.

The comparing step may be carried out by any suitable means, such as by calculating a Combination Index, where a value less than 1 (e.g., less than 0.9) indicates the compounds are synergistic. Any tumor cells can be used, including
neuroblastoma, lung, melanoma, prostate, leukemia, colon, breast, and pancreas tumor cells. Any ceramide-generating retinoid such as fenretinide can be used. Other hyperproliferative cells including pre-malignant and non-malignant cells can be used instead of tumor cells, as noted with respect to conditions for treatment above. In preferred embodiments, the test compound is a ceramide-degradation inhibitor, or other agent that manipulates cellular metabolism or cellular control of ceramide-generated cytotoxicity. The determining step may be carried out by looking for growth inhibition or cytotoxicity in general, or by particularly determining necrosis, apoptosis, or both. The method may be used to identify active compounds that are ceramide-degradation inhibitors, other compounds that manipulate cellular metabolism or cellular control of ceramide-generated cytotoxicity, or compounds that operate by still other mechanisms in addition to those described herein.

Compounds (including the pharmaceutically acceptable salts thereof) that have not previously been known as useful in a method of treating hyperproliferative diseases in combination with a ceramide-generating retinoid, can be prepared, formulated and used in the methods described herein in addition to, or in alternative to, the ceramide-degradation inhibitors described herein. Depending upon the compounds selected for screening, such compounds may be novel compounds, may be known compounds but not previously known for a medicinal or pharmaceutical use, may be compounds previously known for a medicinal or pharmaceutical use but not previously known for use in combination with a ceramide-generating retinoid as described herein.

### 4. Formulations and Administration.

The active compounds described above may be formulated for administration in a single pharmaceutical carrier or in separate pharmaceutical carriers for the treatment of a variety of conditions. In the manufacture of a pharmaceutical formulation according to the invention, the active compounds including the physiologically acceptable salts thereof, or the acid derivatives of either thereof are typically admixed with, inter alia, an acceptable carrier. The carrier must, of course, be acceptable in the sense of being compatible with any other ingredients in the formulation and must not be deleterious to the patient. The carrier may be a solid or a liquid, or both, and is preferably formulated with the compound as a unit-dose formulation, for example, a tablet, which may contain from 0.5% to 95% by weight of the active compound. One or more active compounds may be incorporated in the formulations of the invention, which may be prepared by any of the well known techniques of pharmacy consisting essentially of admixing the components, optionally including one or more accessory ingredients.

The formulations of the invention include those suitable for oral, rectal, topical, buccal (e.g., sub-lingual), vaginal, parenteral (e.g., subcutaneous, intramuscular, intradermal, or intravenous), topical (i.e., both skin and mucosal surfaces, including airway surfaces) and transdermal administration, although the most suitable route in any given case will depend on the nature and severity of the condition being treated and on the nature of the particular active compound which is being used.

Formulations suitable for oral administration may be presented in discrete units, such as capsules, cachets, lozenges, or tablets, each containing a predetermined amount of the active compound; as a powder or granules; as a solution or a suspension in an aqueous or non-aqueous liquid; or as an oil-in-water or water-in-oil emulsion. Such formulations may be prepared by any suitable method of pharmacy which includes the step of bringing into association the active compound and a suitable carrier (which may contain one or more accessory ingredients as noted above). In general, the formulations of the invention are prepared by uniformly and intimately admixing the active compound with a liquid or finely divided solid carrier, or both, and then, if necessary, shaping the resulting mixture. For example, a tablet may be prepared by compressing or molding a powder or granules containing the active compound, optionally with one or more accessory ingredients. Compressed tablets may be prepared by compressing, in a suitable machine, the compound in a free-flowing form, such as a powder or granules optionally mixed with a binder, lubricant, inert diluent, and/or surface active/dispersing agent(s). Molded tablets may be made by molding, in a suitable machine, the powdered compound moistened with an inert liquid binder.

Formulations suitable for buccal (sub-lingual) administration include lozenges comprising the active compound in a flavoured base, usually sucrose and acacia or tragacanth; and pastilles comprising the compound in an inert base such as gelatin and glycerin or sucrose and acacia.

Formulations of the present invention suitable for parenteral or vaginal administration conveniently comprise sterile aqueous preparations of the active compound, which preparations are preferably isotonic with the blood of the intended recipient. These preparations may be administered by means of subcutaneous, intravenous, intramuscular, or intradermal injection. Such preparations may conveniently be prepared by admixing the compound with water or a glycine buffer and rendering the resulting solution sterile and isotonic with the blood.

Formulations suitable for rectal administration are preferably presented as unit dose suppositories. These may be prepared by admixing the active compound with one or more conventional solid carriers, for example, cocoa butter, and then shaping the resulting mixture.

Formulations suitable for topical application to the skin preferably take the form of an ointment, cream, lotion, paste, gel, spray, aerosol, or oil. Carriers which may be used include vaseline, lanoline, polyethylene glycols, alcohols, transdermal enhancers, and combinations of two or more thereof.

Formulations suitable for transdermal administration may be presented as discrete patches adapted to remain in intimate contact with the epidermis of the recipient for a prolonged period of time. Formulations suitable for transdermal administration may also be delivered by iontophoresis (see, for example, Pharmaceutical Research 3 (6):318 (1986)) and typically take the form of an optionally buffered aqueous solution of the active compound. Suitable formulations comprise citrate or bistris buffer (pH 6) or ethanol/water and contain from 0.1 to 0.2M active ingredient.

The compounds for use, pharmaceutical compositions and medicaments for use of the present
invention, comprising the active compounds (including the pharmaceutically acceptable salts
thereof), may be provided in pharmaceutically acceptable carriers for oral, rectal, topical, buccal, parenteral, intramuscular, intradermal, or intravenous, and transdermal administration.

The therapeutically effective dosage of any one active agent
will vary somewhat from compound to compound, patient to patient, and will depend upon factors such as the condition of the patient and the route of delivery. Such dosages can be determined in accordance with routine pharmacological procedures known to those skilled in the art, particularly in light of the disclosure provided herein.

For fenretinide, for systemic treatment, a dose to achieve a plasma level of about 1, 2, or 3 .mu.M to 10 or 20 .mu.M will be employed; typically (for oral dosing) 50 or 100 to 500 or 1000, 2000 or 3000 mg/m.sup.2 body surface area per day.

The present invention is explained in greater detail in the following general examples, followed by more specific examples.

### EXAMPLES A1 - A26

**Cytotoxicity Assay**: Cytotoxicity in cell lines was determined using a fluorescence-based assay employing digital imaging microscopy (DIMSCAN)(after Fragala, et al, Mol Cancer Ther, 6:886-897, 2007). DIMSCAN quantitates viable cells which selectively accumulate fluorescein diacetate and is capable of measuring cytotoxicity over a 4 - 5 log dynamic range by quantifying total fluorescence per well (which is proportional to viable, clonogenic cells) after eliminating background fluorescence using digital thresholding and eosin Y quenching. Briefly, cell lines were seeded into 96-well plates in 100 L of complete culture medium (10 - 20% serum) per well. Cells were incubated overnight in 2%, 5% or 20% oxygen (room air) prior to addition of drugs in 50 L volumes of complete medium to various final drug concentrations in replicates of 12 wells per concentration. In assays employing D-threo-PPMP, PPMP was employed at a fixed, minimally-toxic, final concentration of 10 µM in each well. Control wells received ethanol (final concentration = 0.12% - 0.20%) in complete medium equivalent to the maximum final ethanol concentration of drug-treated wells. Plates were incubated at various oxygen concentrations to simulate physiological hypoxia (i.e., 2% oxygen = typical solid tumor oxygen levels; 5% = bone marrow oxygen levels for leukemias; 20% room air = supraphysiological oxygen for comparison with typical laboratory culture conditions). Plates were assayed at 3 - 4 days after initiating drug exposure depending on the growth properties of each cell line, to allow for maximum cell death and outgrowth of surviving cells. To measure cytotoxicity, FDA (stock solution of 1 mg/ml in DMSO) was added, in 50 L of complete medium per well, to a final concentration of 10 g/ml. The plates were incubated for an additional 15 - 30 minutes at 37 C and then 30 L of eosin Y (0.5% in normal saline) was added per well. Total fluorescence of each well was then measured using digital imaging microscopy.

**FIGS. 1** to **26** illustrate the effect (Examples A1 through A26) of the claimed invention and were carried out using the procedures disclosed herein.
**FIG. 1** is the dose-response of C17-L-threo-sphinganine in combination with fenretinide. Assayed by DimScan methodology at +96 hrs. Synergy assessed by the Combination Index Method of Chou, et al. C17-L-threo-sphinganine synergized (C.I. < 1) fenretinide cytotoxicity at one or more doses in the drug-resistant CHLA-90 neuroblastoma cancer cell line;
**FIG. 2** is the dose-response of C17-L-threo-sphinganine in combination with fenretinide. Assayed by DimScan methodology at +96 hrs. Synergy assessed by the Combination Index Method of Chou, et al. C17-L-threo-sphinganine synergized (C.I. < 1) fenretinide cytotoxicity at one or more doses in the CHLA-266 brain cancer (PNET) cell line in 2% oxygen;

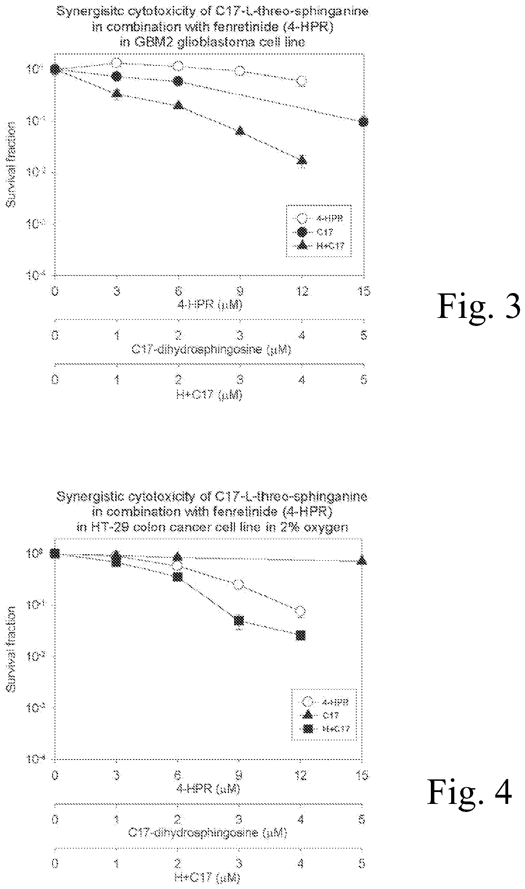

**FIG. 3** is the dose-response of C17-L-threo-sphinganine in combination with fenretinide. Assayed by DimScan methodology at +96 hrs. Synergy assessed by the Combination Index Method of Chou, et al. C17-L-threo-sphinganine synergized (C.I. < 1) fenretinide cytotoxicity at one or more doses in the GBM2 glioblastoma brain cancer cell line;
**FIG. 4** is the dose-response of C17-L-threo-sphinganine in combination with fenretinide. Assayed by DimScan methodology at +96 hrs. Synergy assessed by the Combination Index Method of Chou, et al. C17-L-threo-sphinganine synergized (C.I. < 1) fenretinide cytotoxicity at one or more doses in the HT-29 colon cancer cell line in 2% oxygen;

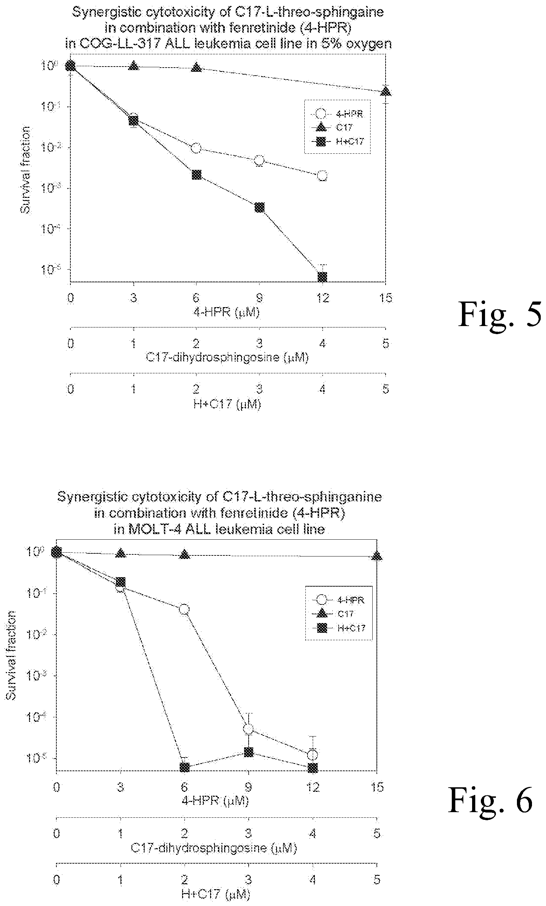

**FIG. 5** is the dose-response of C17-L-threo-sphinganine in combination with fenretinide. Assayed by DimScan methodology at +96 hrs. Synergy assessed by the Combination Index Method of Chou, et al. C17-L-threo-sphinganine synergized (C.I. < 1) fenretinide cytotoxicity at one or more doses in the COG-LL-317 Acute Lymphoblastic Leukemia (ALL) cancer cell line in 5% oxygen;
**FIG. 6** is the dose-response of C17-L-threo-sphinganine in combination with fenretinide. Assayed by DimScan methodology at +96 hrs. Synergy assessed by the Combination Index Method of Chou, et al. C17-L-threo-sphinganine synergized (C.I. < 1) fenretinide cytotoxicity at one or more doses in the MOLT-4 ALL leukemia cell line;

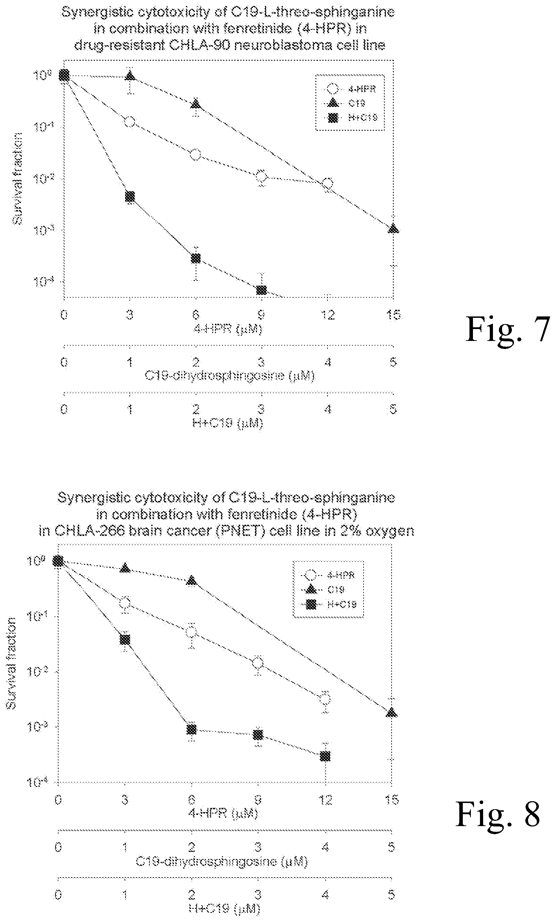

**FIG. 7** is the dose-response of C19-L-threo-sphinganine in combination with fenretinide. Assayed by DimScan methodology at +96 hrs. Synergy assessed by the Combination Index Method of Chou, et al. C19-L-threo-sphinganine synergized (C.I. < 1) fenretinide cytotoxicity at one or more doses in drug resistant the CHLA-90 neuroblastoma cancer cell line;
**FIG. 8** is the dose-response of C19-L-threo-sphinganine in combination with fenretinide. Assayed by DimScan methodology at +96 hrs. Synergy assessed by the Combination Index Method of Chou, et al. C19-L-threo-sphinganine synergized (C.I. < 1) fenretinide cytotoxicity at one or more doses in the CHLA-266 brain cancer (PNET) cell line in 2% oxygen;

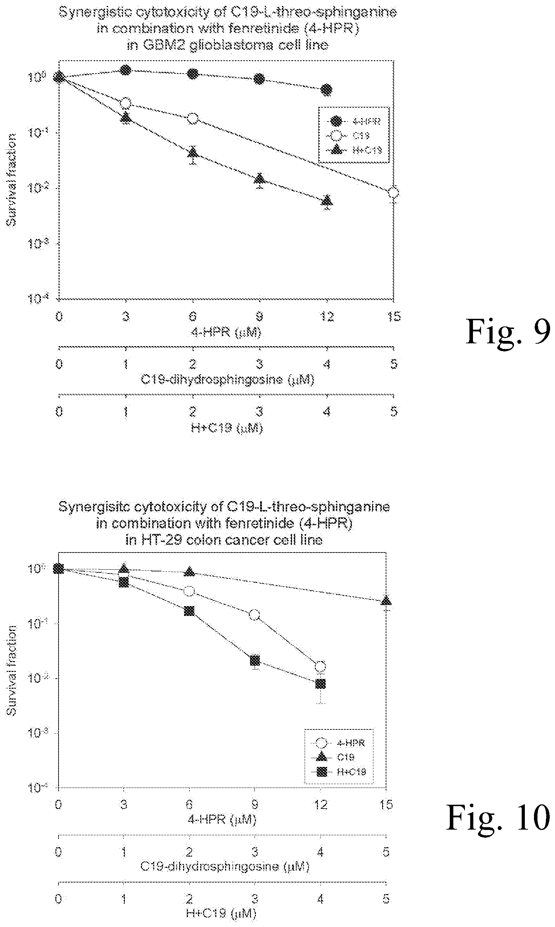

**FIG. 9** is the dose-response of C19-L-threo-sphinganine in combination with fenretinide. Assayed by DimScan methodology at +96 hrs. Synergy assessed by the Combination Index Method of Chou, et al. C19-L-threo-sphinganine synergized (C.I. < 1) fenretinide cytotoxicity at one or more doses in the GBM2 glioblastoma brain cancer cell line;
**FIG. 10** is the dose-response of C19-L-threo-sphinganine in combination with fenretinide. Assayed by DimScan methodology at +96 hrs. Synergy assessed by the Combination Index Method of Chou, et al. C19-L-threo-sphinganine synergized (C.I. < 1) fenretinide cytotoxicity at one or more doses in the HT-29 colon cancer cell line;

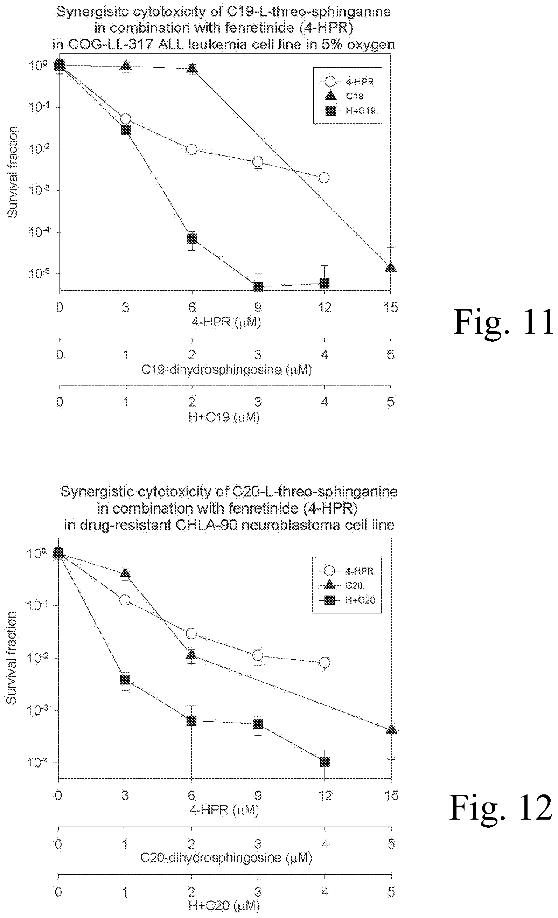

**FIG. 11** is the dose-response of C19-L-threo-sphinganine in combination with fenretinide. Assayed by DimScan methodology at +96 hrs. Synergy assessed by the Combination Index Method of Chou, et al. C19-L-threo-sphinganine synergized (C.I. < 1) fenretinide cytotoxicity at one or more doses in the COG-LL-317 ALL leukemia cell line in 5% oxygen;
**FIG. 12** is the dose-response of C20-L-threo-sphinganine in combination with fenretinide. Assayed by DimScan methodology at +96 hrs. Synergy assessed by the Combination Index Method of Chou, et al. C20-L-threo-sphinganine synergized (C.I. < 1) fenretinide cytotoxicity at one or more doses in the drug resistant CHLA-90 neuroblastoma cell line;

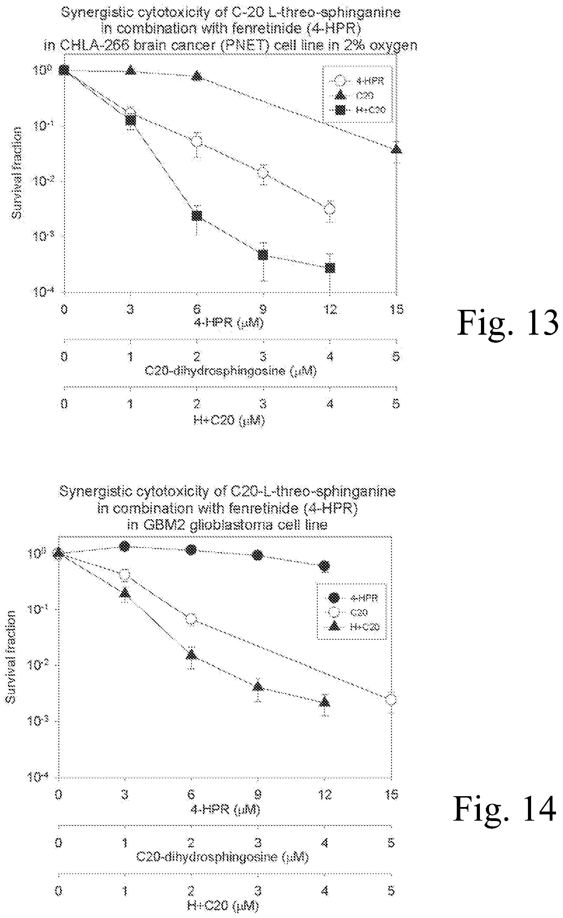

**FIG. 13** is the dose-response of C20-L-threo-sphinganine in combination with fenretinide. Assayed by DimScan methodology at +96 hrs. Synergy assessed by the Combination Index Method of Chou, et al. C20-L-threo-sphinganine synergized (C.I. < 1) fenretinide cytotoxicity at one or more doses in the CHLA-266 brain cancer (PNET) cell line in 2% oxygen;
**FIG. 14** is the dose-response of C20-L-threo-sphinganine in combination with fenretinide. Assayed by DimScan methodology at +96 hrs. Synergy assessed by the Combination Index Method of Chou, et al. C20-L-threo-sphinganine synergized (C.I. < 1) fenretinide cytotoxicity at one or more doses in the GBM2 glioblastoma brain cancer cell line;

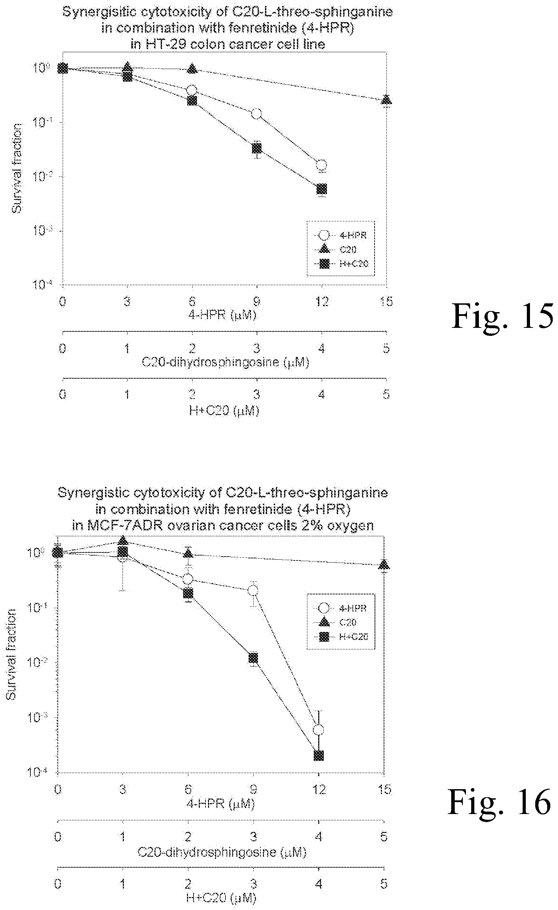

**FIG. 15** is the dose-response of C20-L-threo-sphinganine in combination with fenretinide. Assayed by DimScan methodology at +96 hrs. Synergy assessed by the Combination Index Method of Chou, et al. C20-L-threo-sphinganine synergized (C.I. < 1) fenretinide cytotoxicity at one or more doses in the HT-29 colon cancer cell line;
**FIG. 16** is the dose-response of C20-L-threo-sphinganine in combination with fenretinide. Assayed by DimScan methodology at +96 hrs. Synergy assessed by the Combination Index Method of Chou, et al. C20-L-threo-sphinganine synergized (C.I. < 1) fenretinide cytotoxicity at one or more doses in the MCF-7/ADR (OVCAR-8/ADR) ovarian cancer cell line in 2% oxygen;

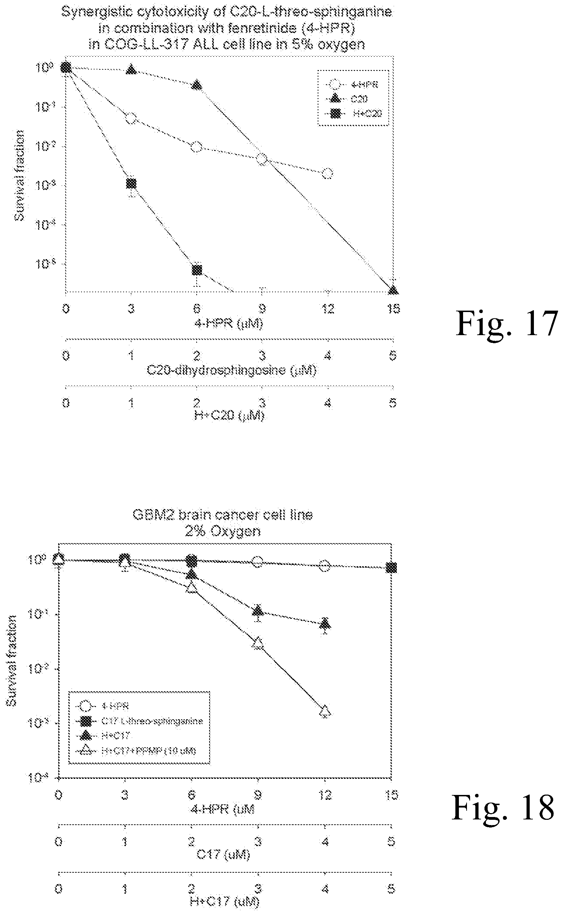

**FIG. 17** is the dose-response of C20-L-threo-sphinganine in combination with fenretinide. Assayed by DimScan methodology at +96 hrs. Synergy assessed by the Combination Index Method of Chou, et al. C20-L-threo-sphinganine synergized (C.I. < 1) fenretinide cytotoxicity at one or more doses in the COG-LL-317 ALL cell line in 5% oxygen;
**FIG. 18** is the dose-response of C17-L-threo-sphinganines in combination with fenretinide and D-threo-PPMP. Assayed by DimScan methodology at +96 hrs. Three drug combination used a fixed, minimally-toxic, concentration of D-threo-PPMP (10 uM), an inhibitor of glucosylceramide synthase and sphingomyelin synthase. PPMP synergistically increased cytotoxicity (C.I. <1) of fenretinide + L-threo-sphinganine at most doses in the GBM2 brain cancer cell line in 2% oxygen by Combination Index Analysis Method of Chou, et al.;

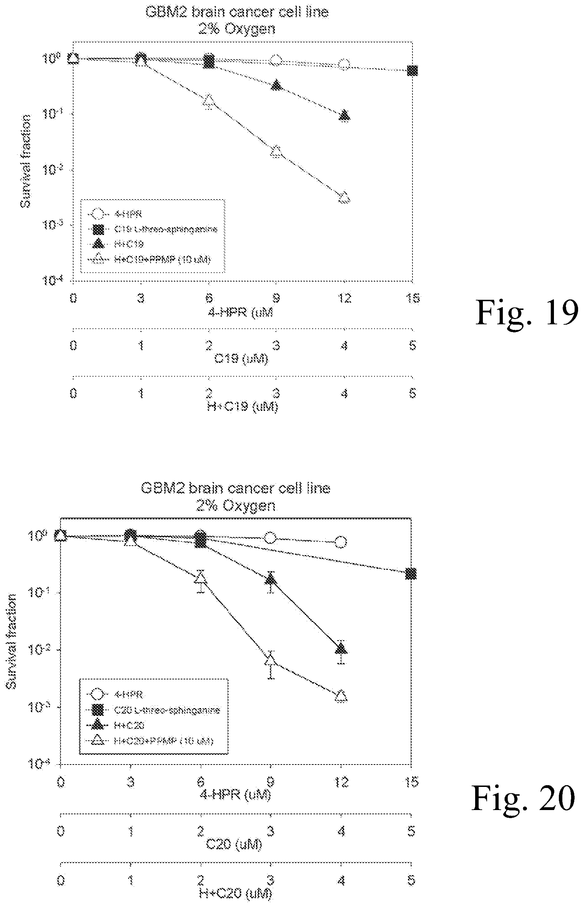

**FIG. 19** is the dose-response of C 19-L-threo-sphinganines in combination with fenretinide and D-threo-PPMP. Assayed by DimScan methodology at +96 hrs. Three drug combination used a fixed, minimally-toxic, concentration of D-threo-PPMP (10 uM), an inhibitor of glucosylceramide synthase and sphingomyelin synthase. PPMP synergistically increased cytotoxicity (C.I. <1) of fenretinide + L-threo-sphinganine at most doses in the GBM2 brain cancer cell line in 2% oxygen by Combination Index Analysis Method of Chou, et al.;
**FIG. 20** is the dose-response of C20-L-threo-sphinganines in combination with fenretinide and D-threo-PPMP. Assayed by DimScan methodology at +96 hrs. Three drug combination used a fixed, minimally-toxic, concentration of D-threo-PPMP (10 uM), an inhibitor of glucosylceramide synthase and sphingomyelin synthase. PPMP synergistically increased cytotoxicity (C.I. <1) of fenretinide + L-threo-sphinganine at most doses in the GBM2 brain cancer cell line in 2% oxygen by Combination Index Analysis Method of Chou, et al.;

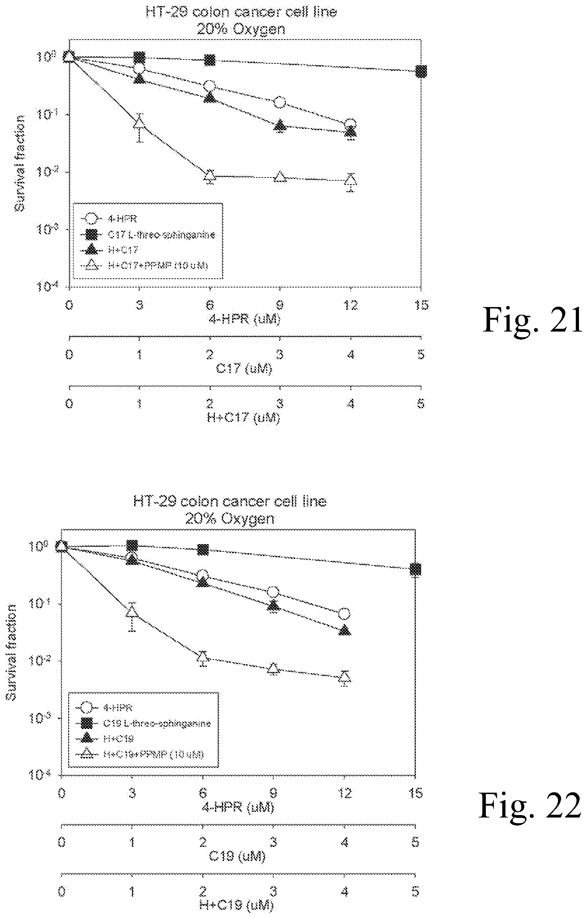

**FIG. 21** is the dose-response of C17-L-threo-sphinganines in combination with fenretinide and D-threo-PPMP. Assayed by DimScan methodology at +96 hrs. Three drug combination used a fixed, minimally-toxic, concentration of D-threo-PPMP (10 uM), an inhibitor of glucosylceramide synthase and sphingomyelin synthase. PPMP synergistically increased cytotoxicity (C.I. <1) of fenretinide + L-threo-sphinganine at most doses in the HT-29 colon cancer cell line in 20% oxygen by Combination Index Analysis Method of Chou, et al.;
**FIG. 22** is the dose-response of C19-L-threo-sphinganines in combination with fenretinide and D-threo-PPMP. Assayed by DimScan methodology at +96 hrs. Three drug combination used a fixed, minimally-toxic, concentration of D-threo-PPMP (10 uM), an inhibitor of glucosylceramide synthase and sphingomyelin synthase. PPMP synergistically increased cytotoxicity (C.I. <1) of fenretinide + L-threo-sphinganine at most doses in the HT-29 colon cancer cell line in 20% oxygen by Combination Index Analysis Method of Chou, et al.;

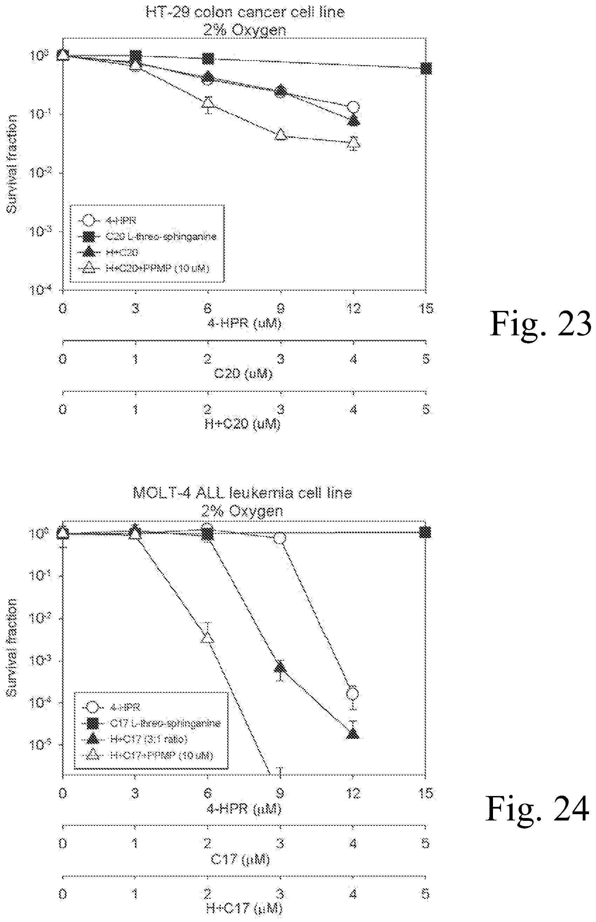

**FIG. 23** is the dose-response of C20-L-threo-sphinganines in combination with fenretinide and D-threo-PPMP. Assayed by DimScan methodology at +96 hrs. Three drug combination used a fixed, minimally-toxic, concentration of D-threo-PPMP (10 uM), an inhibitor of glucosylceramide synthase and sphingomyelin synthase. PPMP synergistically increased cytotoxicity (C.I. <1) of fenretinide + L-threo-sphinganine at most doses in the HT-29 colon cancer cell line in 2% oxygen by Combination Index Analysis Method of Chou, et al.;
**FIG. 24** is the dose-response of C17-L-threo-sphinganines in combination with fenretinide and D-threo-PPMP. Assayed by DimScan methodology at +96 hrs. Three drug combination used a fixed, minimally-toxic, concentration of D-threo-PPMP (10 uM), an inhibitor of glucosylceramide synthase and sphingomyelin synthase. PPMP synergistically increased cytotoxicity (C.I. <1) of fenretinide + L-threo-sphinganine at most doses in the MOLT-4 ALL leukemia cell line in 2% oxygen by Combination Index Analysis Method of Chou, et al.;

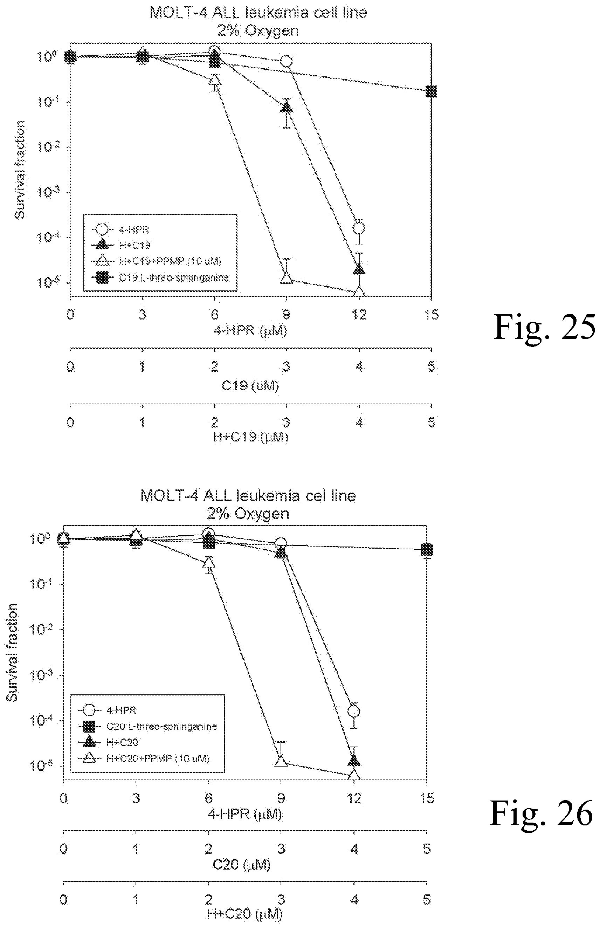

**FIG. 25** is the dose-response of C19-L-threo-sphinganines in combination with fenretinide and D-threo-PPMP. Assayed by DimScan methodology at +96 hrs. Three drug combination used a fixed, minimally-toxic, concentration of D-threo-PPMP (10 uM), an inhibitor of glucosylceramide synthase and sphingomyelin synthase. PPMP synergistically increased cytotoxicity (C.I. <1) of fenretinide + L-threo-sphinganine at most doses in the MOLT-4 ALL leukemia cell line in 2% oxygen by Combination Index Analysis Method of Chou, et al.;
**FIG. 26** is the dose-response of C20-L-threo-sphinganines in combination with fenretinide and D-threo-PPMP. Assayed by DimScan methodology at +96 hrs. Three drug combination used a fixed, minimally-toxic, concentration of D-threo-PPMP (10 uM), an inhibitor of glucosylceramide synthase and sphingomyelin synthase. PPMP synergistically increased cytotoxicity (C.I. <1) of fenretinide + L-threo-sphinganine at most doses in the MOLT-4 ALL leukemia cell line in 2% oxygen by Combination Index Analysis Method of Chou, et al.

### EXAMPLES B1 - B21

**Cytotoxicity Assay** - Once again, cytotoxicity is determined using the DIMSCAN assay system (R. Proffitt et al., Cytomety 24, 204-213 (1996); T. Frgala et al., Mol Cancer Ther. 6:886-97, 2007). The system employs digital imaging microscopy to quantify viable cells, which selectively accumulate fluorescein diacetate to become brightly fluorescent. The system is capable of measuring cytotoxicity over a 4-5 log dynamic range by quenching the residual fluorescence of dead and dying cells with eosin Y and quantifying the total fluorescence of viable cells using digital thresholding. Measured fluorescence is directly proportionate to the number of viable cells. A comparison of the total fluorescence of a drug-treated cell population to the fluorescence of a similar number of untreated cells yields a survival fraction. In brief, 2000 to 10,000 cells/well (depending on size and growth rates) are replicate plated into 60 wells of a 96-well tissue culture plate in 0.1 mL of whole medium and allowed to attach overnight. Drug(s) are then added in 0.05 mL of whole medium to the final concentrations indicated. Drugs (fenretinide, and various chain length L-*threo*-sphinganines) were tested both as single agents and in a 3:1 ratio of fenretinide:sphinganine. C18-L-*threo*-sphinganine, or "safingol,"
as well as C12 and C14-L-*threo*-sphinganine were also tested and results are shown for reference. There are 12 wells treated per drug concentration. Twelve wells receive drug vehicle-only to the appropriate final concentration and serve as controls for the plate. Cells are incubated for 96 hours at 37°C in ambient air with 5% CO₂. Fluorescein diacetate is then added to each well in 0.05 mL media to a final concentration of 8 microgram/mL. Cells are incubated for a further 15 minutes at 37°C and 0.03 mL of 0.5% eosin Y is added to each well. Total fluorescence of viable cells is then measured by digital imaging microscopy. Results were graphed as the ratio of the fluoresecence of drug-treated wells to non-drug treated control wells (i.e., Survival Fraction). All tests were performed at least twice. Representative results are shown.

Examples also shows tables containing the Combination Index (CI) calculated using CalcuSyn version 2.0 software, BIOSOFT®, Cambridge, UK, (Chou-Talalay "dose-effect" analysis, Trends Pharmacol. Sci. 4, 450-454, 1983, Chou, Cancer Res; 70:440-446, 2010) as a measure of drug synergy for the L-*threo*-sphinganines tested with fenretinide. The Combination Index (CI) is a term to describe mathematical modeling of the pharmacologic effects on cytotoxicity (cell death) of two drugs in combination based. Cytotoxic "synergy" is defined as a cell death affect which is greater than would be expected from a simple product of the single agent cytotoxicities alone (i.e. an "additive" effect). By Chou-Talalay analysis, a CI < 0.9 on a Fraction Affected ("Fₐ", i.e. fraction of cells killed) indicates synergy, with smaller numbers indicating greater synergy; a CI of 0.9 - 1.1 signifies an additive or near-additive effect; and a CI > 1.1 means the drug combination is antagonistic.

It is notable that multi-log cytotoxicity was achieved in cell lines that were p53 null or mutant and in cell lines that are highly resistant to alkylating agents.

Results demonstrate that, while not all are equally active in all human cancer cell lines, all non-C18-L-*threo*-sphinganines can increase fenretinide cytotoxicity, either additively or synergistically, in a broad range of human cancer cell types, including both solid and hematopoietic cancers, and adult and pediatric cancers.

### EXAMPLE B1

Fenretinide and L-*threo*-sphinganines tested in normal human fibroblast (normal skin cell) cell lines, CRL-2091 and CRL-2076. Results show that L-*threo-*sphinganines and fenretinide combinations are minimally cytotoxic in normal human cell. Reference is made to the graphs plotting the results shown in **FIGS**. **27A - 27D** which results are reported in **TABLES 1.1** and **1.2** below.

**TABLE 1.1**

| CRL-2091 20% O2 | | | | | | | | | | | | | | | | | |
|---|---|---|---|---|---|---|---|---|---|---|---|---|---|---|---|---|---|
| 4-HPR | L-threo-Sphinganine | C12 | | C14 | | C15 | | C17 | | **C18** | | C19 | | C20 | | C21 | |
| (µm) | (µm) | Fₐ | Cl | Fₐ | Cl | Fₐ | Cl | Fₐ | Cl | Fₐ | Cl | Fₐ | Cl | Fₐ | Cl | Fₐ | Cl |
| 1.5 | 0.5 | 0.27 | >1 | | | | | 0.32 | >1 | 0.26 | >1 | 0.13 | >1 | 0.16 | >1 | | |
| 3 | 1 | 0.44 | 0.76 | | | | | 0.40 | >1 | 0.39 | >1 | 0.25 | >1 | 0.29 | >1 | | |
| 6 | 2 | 0.42 | >1 | | | | | 0.21 | >1 | 0.29 | >1 | 0.13 | >1 | 0.29 | >1 | | |
| 9 | 3 | 0.54 | >1 | | | | | 0.69 | >1 | 0.66 | >1 | 0.62 | >1 | 0.73 | 0.33 | | |

**TABLE 1.2**

| CRL-2076, 20% O₂ | | | | | | | | | | | | | | | | | |
|---|---|---|---|---|---|---|---|---|---|---|---|---|---|---|---|---|---|
| 4-HPR | L-threo-Sphinganine | C12 | | C14 | | C15 | | C17 | | **C18** | | C19 | | C20 | | C21 | |
| (µm) | (µM) | Fₐ | Cl | Fₐ | Cl | Fₐ | Cl | Fₐ | Cl | Fₐ | Cl | Fₐ | Cl | Fₐ | Cl | Fₐ | Cl |
| 1.5 | 0.5 | 0.17 | 1 | | | | | 0.10 | >1 | **0.08** | 1 | 0.05 | >1 | 0.20 | >1 | | |
| 3 | 1 | 0.30 | >1 | | | | | 0.10 | >1 | **0.09** | >1 | 0.04 | >1 | 0.11 | 1 | | |
| 6 | 2 | 0.29 | >1 | | | | | 0.12 | >1 | **0.06** | >1 | 0.09 | >1 | 0.35 | >1 | | |
| 9 | 3 | 0.30 | >1 | | | | | 0.08 | >1 | **0.07** | >1 | 0.15 | >1 | 0.94 | >1 | | |

### EXAMPLE B2

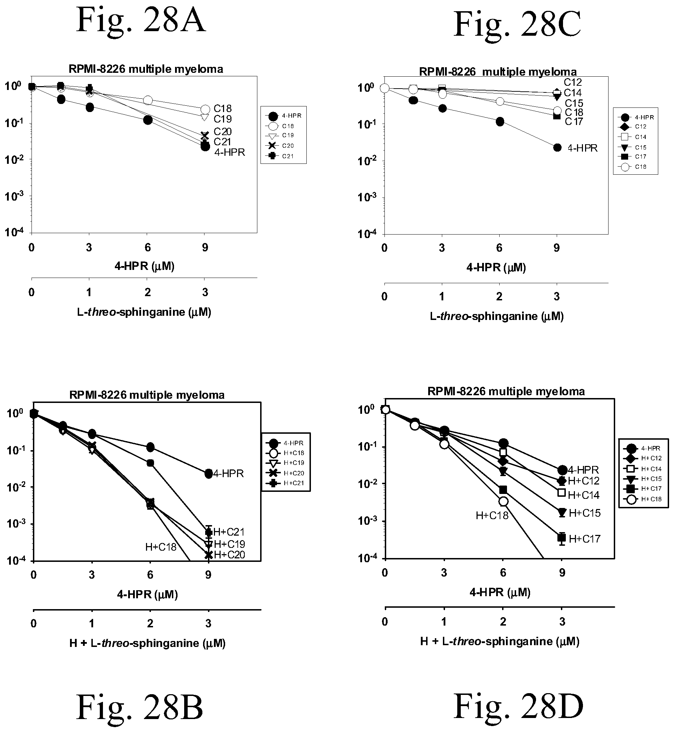

Fenretinide and L-*threo*-sphinganines tested in human Multiple Myeloma (a cancer of the blood and bone marrow) cell line, RPMI-8226. Results show that at least some L-*threo*-sphinganines increased fenretinide cytotoxicity, additively or synergistically, depending on the drug concentrations tested. Reference is made to the graphs plotting the results shown in **FIGS. 28A - 28D** which results are reported in **TABLE 2.1** below.

**TABLE 2.1**

| RPMI-8226 | | | | | | | | | | | | | | | | | |
|---|---|---|---|---|---|---|---|---|---|---|---|---|---|---|---|---|---|
| 4-HPR | L-threo-Sphinganine | C12 | | C14 | | C15 | | C17 | | **C18** | | C19 | | C20 | | C21 | |
| (µm) | (µM) | Fₐ | Cl | Fₐ | Cl | Fₐ | Cl | Fₐ | Cl | Fₐ | Cl | Fₐ | Cl | Fₐ | Cl | Fₐ | Cl |
| 1.5 | 0.5 | 0.6 | 0.8 | 0.606 | 0.8 | 0.518 | 1.0 | 0.549 | 1.12 | 0.616 | 1.0 | 0.649 | 0.9 | 0.583 | 1.0 | 0.517 | 1.2 |
| 3 | 1 | 0.757 | 1.0 | 0.747 | 1.1 | 0.75 | 1.1 | 0.859 | 1.00 | 0.879 | 0.9 | 0.895 | 0.9 | 0.867 | 1.1 | 0.722 | 1.6 |
| 6 | 2 | 0.96 | 0.7 | 0.93 | 1.0 | 0.978 | 0.5 | 0.993 | 0.43 | 0.997 | 0.2 | 0.996 | 0.5 | 0.996 | 0.9 | 0.955 | 1.6 |
| 9 | 3 | 0.988 | 0.5 | 0.994 | 0.4 | 0.998 | 0.2 | 1.000 | >0.1 | 1 | >0.1 | 1.000 | >0.1 | 1 | 0.3 | 0.999 | 1.1 |

### EXAMPLE B3

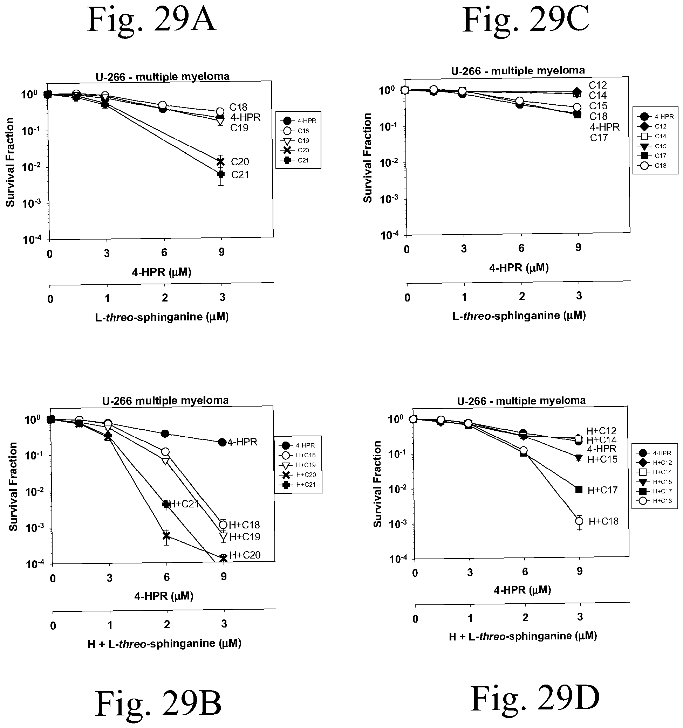

Fenretinide and L-*threo*-sphinganines tested in human Multiple Myeloma (a cancer of the blood and bone marrow) cell line, U-266. Results show that at least some L-*threo*-sphinganines increased fenretinide cytotoxicity, additively or synergistically, depending on the drug concentrations tested. Reference is made to the graphs plotting the results shown in **FIGS. 29A - 29D** which results are reported in **TABLE 3.1** below.

**TABLE 3.1**

| U-266 | | | | | | | | | | | | | | | | | |
|---|---|---|---|---|---|---|---|---|---|---|---|---|---|---|---|---|---|
| 4-HPR | L-threo-Sphinganine | C12 | | C14 | | C15 | | C17 | | **C18** | | C19 | | C20 | | C21 | |
| (µM) | (µM) | Fₐ | Cl | Fₐ | Cl | Fₐ | Cl | Fₐ | Cl | Fₐ | Cl | Fₐ | Cl | Fₐ | Cl | Fₐ | Cl |
| 1.5 | 0.5 | 0.176 | 0.8 | 0.163 | 0.9 | 0.085 | 1.2 | 0.134 | 1.3 | 0.048 | 1.4 | 0.19 | 1.0 | 0.259 | 1.2 | 0.225 | 1.4 |
| 3 | 1 | 0.326 | 0.9 | 0.315 | 1.1 | 0.241 | 1.5 | 0.35 | 1.5 | 0.268 | 1.5 | 0.414 | 1.4 | 0.688 | 1.3 | 0.658 | 1.5 |
| 6 | 2 | 0.697 | 0.9 | 0.687 | 1.0 | 0.697 | 1.2 | 0.901 | 0.9 | 0.883 | 1.3 | 0.933 | 0.9 | 0.999 | 0.4 | 0.996 | 0.6 |
| 9 | 3 | 0.748 | 1.2 | 0.76 | 1.3 | 0.929 | 0.9 | 0.991 | 0.4 | 0.999 | 0.8 | 0.999 | 0.3 | 1.000 | >0.1 | 1 | >0.1 |

### EXAMPLE B4

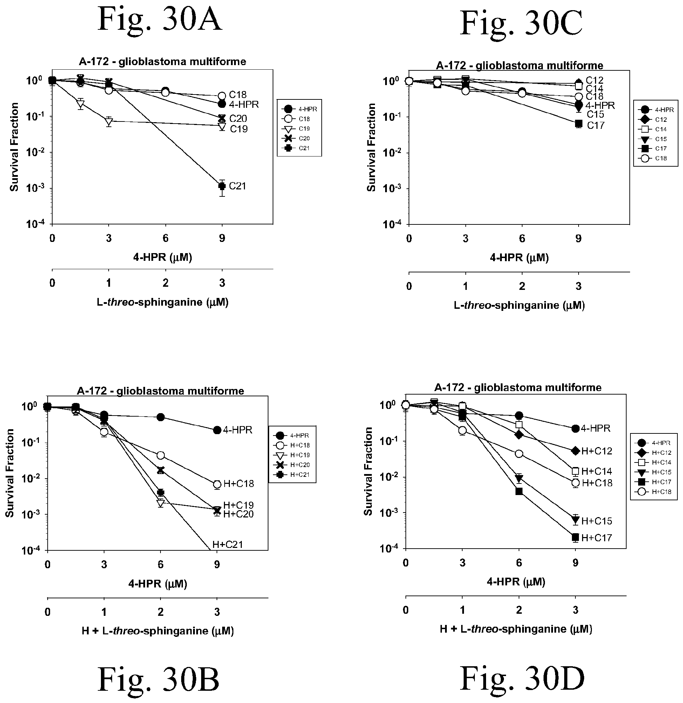

Fenretinide and L-*threo*-sphinganines tested in human Glioblastoma multiforme (brain cancer) cell line, A-172. Results show that at least some L-*threo-*sphinganines increased fenretinide cytotoxicity, additively or synergistically, depending on the drug concentrations tested. Reference is made to the graphs plotting the results shown in **FIGS. 30A - 30D** which results are reported in **TABLE 4.1** below.

**TABLE 4.1**

| A-172 | | | | | | | | | | | | | | | | | |
|---|---|---|---|---|---|---|---|---|---|---|---|---|---|---|---|---|---|
| 4-HPR | L-threo-Sphinganine | C12 | | C14 | | C15 | | C17 | | **C18** | | C19 | | C20 | | C21 | |
| (µm) | (µm) | Fₐ | Cl | Fₐ | Cl | Fₐ | Cl | Fₐ | Cl | **Fₐ** | **Cl** | Fₐ | Cl | Fₐ | Cl | Fₐ | Cl |
| 1.5 | 0.5 | 0.024 | 6.1 | 0.001 | 23.7 | 0.001 | 23.7 | 0.232 | 1.4 | 0.233 | 1.3 | 0.086 | 84.4 | 0.039 | 2.8 | 0.006 | 8.8 |
| 3 | 1 | 0.065 | 4.4 | 0.065 | 3.8 | 0.378 | 1.4 | 0.529 | 1.4 | 0.803 | 0.5 | 0.578 | 8.1 | 0.594 | 1.0 | 0.537 | 1.6 |
| 6 | 2 | 0.849 | 0.4 | 0.717 | 1.0 | 0.991 | 0.4 | 0.996 | 0.2 | 0.956 | 0.3 | 0.998 | >0.1 | 0.983 | 0.6 | 0.996 | 0.8 |
| 9 | 3 | 0.981 | 0.2 | 0.958 | 0.5 | 0.998 | 0.4 | 1.000 | >0.1 | 0.993 | 0.1 | 1.000 | >0.1 | 1.000 | >0.1 | 1.000 | >0.1 |

### EXAMPLE B5

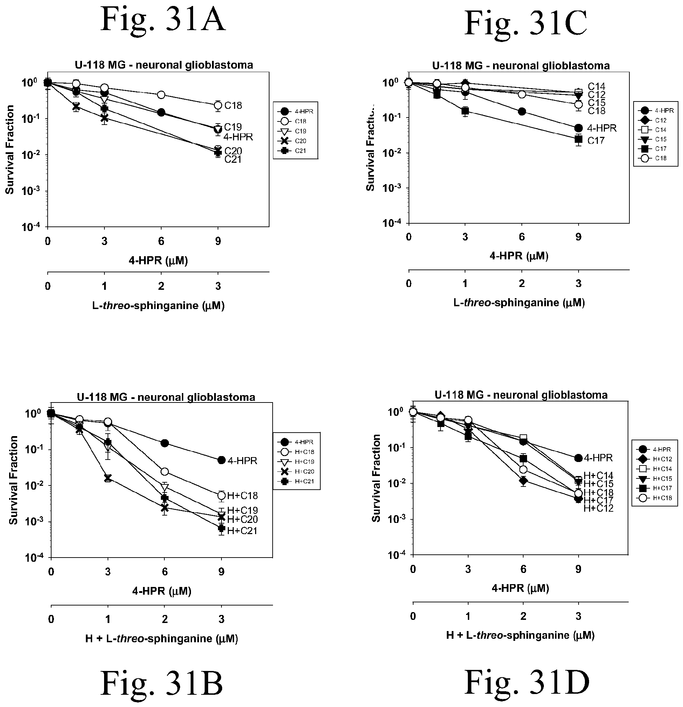

Fenretinide and L-*threo*-sphinganines tested in human Glioblastoma (brain cancer) cell line, U-118. Results show that at least some L-*threo*-sphinganines increased fenretinide cytotoxicity, additively or synergistically, depending on the drug concentrations tested. Reference is made to the graphs plotting the results shown in **FIGS. 31A - 31D** which results are reported in **TABLE 5.1** below.

**TABLE 5.1**

| U-118 MG | | | | | | | | | | | | | | | | | |
|---|---|---|---|---|---|---|---|---|---|---|---|---|---|---|---|---|---|
| 4-HPR | L-threo-Sphinganine | C12 | | C14 | | C15 | | C17 | | **C18** | | C19 | | C20 | | C21 | |
| (µM) | (µM) | Fₐ | Cl | Fₐ | Cl | Fₐ | Cl | Fₐ | Cl | Fₐ | Cl | Fₐ | Cl | Fₐ | Cl | Fₐ | Cl |
| 1.5 | 0.5 | 0.226 | 1.5 | 0.318 | 1.4 | 0.371 | 0.4 | 0.519 | 1.7 | 0.317 | 1.4 | 0.530 | 1.3 | 0.645 | 1.8 | 0.588 | 1.3 |
| 3 | 1 | 0.671 | 1.0 | 0.586 | 1.2 | 0.577 | 0.5 | 0.791 | 1.8 | 0.407 | 2.3 | 0.882 | 0.9 | 0.984 | 0.5 | 0.836 | 1.5 |
| 6 | 2 | 0.988 | 0.3 | 0.814 | 1.2 | 0.847 | 0.4 | 0.951 | 1.5 | 0.976 | 0.5 | 0.991 | 0.4 | 0.998 | 0.3 | 0.995 | 0.6 |
| 9 | 3 | 0.996 | 0.2 | 0.988 | 0.4 | 0.989 | 0.1 | 0.995 | 0.7 | 0.995 | 0.3 | 0.998 | 0.3 | 0.999 | 0.3 | 0.999 | 0.5 |

### EXAMPLE B6

Fenretinide and L-*threo*-sphinganines tested in human Glioblastoma multiforme (brain cancer) cell line, T98G. Results show that at least some L-*threo-*sphinganines increased fenretinide cytotoxicity, additively or synergistically, depending on the drug concentrations tested. Reference is made to the graphs plotting the results shown in **FIGS. 32A - 32D** which results are reported in **TABLE 6.1** below.

**TABLE 6.1**

| T98G | | | | | | | | | | | | | | | | | |
|---|---|---|---|---|---|---|---|---|---|---|---|---|---|---|---|---|---|
| 4-HPR | L-threo-Sphinganine | C12 | | C14 | | C15 | | C17 | | **C18** | | C19 | | C20 | | C21 | |
| (µM) | (µM) | Fₐ | Cl | Fₐ | Cl | Fₐ | Cl | Fₐ | Cl | Fₐ | Cl | Fₐ | Cl | Fₐ | Cl | Fₐ | Cl |
| 1.5 | 0.5 | 0.306 | 2.8 | 0.544 | 1.0 | 0.516 | 1.3 | 0.447 | 1.7 | 0.657 | 0.786 | 0.584 | 0.9 | 0.397 | 2.4 | 0.574 | 1.2 |
| 3 | 1 | 0.408 | 3.6 | 0.602 | | 0.683 | 1.3 | 0.887 | 0.3 | 0.701 | 1.316 | 0.704 | 1.1 | 0.989 | 0.2 | 0.805 | 1.0 |
| 6 | 2 | 0.781 | 1.5 | 0.839 | 1.6 1.0 | 0.849 | 1.0 | 0.944 | 0.3 | 0.906 | 0.767 | 0.990 | 0.1 | 1.000 | >0.1 | 0.832 | 1.8 |
| 9 | 3 | 0.981 | 0.2 | 0.941 | 0.5 | 0.962 | 0.4 | 0.980 | 0.2 | 0.999 | 0.023 | 0.995 | 0.1 | 1.000 | >0.1 | 0.999 | 0.1 |

### EXAMPLE B7

Fenretinide and L-*threo*-sphinganines tested in human Glioblastoma multiforme (brain cancer) cell line, SJ-GBM2. Results show that at least some L-*threo-*sphinganines increased fenretinide cytotoxicity, additively or synergistically, depending on the drug concentrations tested. Reference is made to the graphs plotting the results shown in **FIGS**. **33A** - **33D** which results are reported in **TABLE 7.1** below.

**TABLE 7.1**

| SJ-GBM2 | | | | | | | | | | | | | | | | | |
|---|---|---|---|---|---|---|---|---|---|---|---|---|---|---|---|---|---|
| 4-HPR | L-threo-Sphinganine | C12 | | C14 | | C15 | | C17 | | **C18** | | C19 | | C20 | | C21 | |
| (µM) | (µM) | Fₐ | Cl | Fₐ | Cl | Fₐ | Cl | Fₐ | Cl | Fₐ | Cl | Fₐ | Cl | Fₐ | Cl | Fₐ | Cl |
| 1.5 | 0.5 | 0.133 | 0.5 | 0.007 | 5.0 | 0.086 | 1.7 | 0.188 | 0.5 | 0.001 | >>1 | 0.001 | >>1 | 0.055 | 0.5 | 0.131 | 14 |
| 3 | 1 | 0.271 | 0.7 | 0.209 | 1.2 | 0.301 | 1.1 | 0.796 | 0.5 | 0.712 | 0.6 | 0.672 | .04 | 0.737 | 0.6 | 0.470 | 1.9 |
| 6 | 2 | 0.930 | 0.7 | 0.598 | 1.2 | 0.765 | 0.9 | 0.962 | 0.7 | 0.987 | 0.6 | 0.925 | 0.7 | 0.998 | 0.7 | 0.946 | 2.1 |
| 9 | 3 | 0.968 | 0.9 | 0.820 | 1.4 | 0.953 | 1.0 | 0.998 | 0.7 | 0.999 | 0.7 | 0.997 | 0.8 | 1.000 | 0.3 | 0.996 | 2.0 |

### EXAMPLE B8

Fenretinide and L-*threo*-sphinganines tested in human Glioblastoma (brain cancer) cell line, SJ-G2. Results show that at least some L-*threo*-sphinganines increased fenretinide cytotoxicity, additively or synergistically, depending on the drug concentrations tested. Reference is made to the graphs plotting the results shown in **FIGS. 34A - 34D** which results are reported in **TABLE 8.1** below.

**TABLE 8.1**

| SJ-G2 | | | | | | | | | | | | | | | | | |
|---|---|---|---|---|---|---|---|---|---|---|---|---|---|---|---|---|---|
| 4-HPR | L-threo-Sphinganine | C12 | | C14 | | C15 | | C17 | | **C18** | | C19 | | C20 | | C21 | |
| (µM) | (µM) | Fₐ | Cl | Fₐ | Cl | Fₐ | Cl | Fₐ | Cl | Fₐ | Cl | Fₐ | Cl | Fₐ | Cl | Fₐ | Cl |
| 1.5 | 0.5 | 0.199 | 0.8 | 0.103 | 1.3 | 0.135 | 1.1 | 0.462 | 0.6 | 0.437 | 0.7 | 0.389 | 0.7 | 0.408 | 0.9 | 0.118 | 1.5 |
| 3 | 1 | 0.530 | 0.07 | 0.307 | 1.3 | 0.282 | 1.3 | 0.874 | 0.4 | 0.728 | 0.7 | 0.700 | 0.8 | 0.773 | 0.8 | 0.299 | 1.9 |
| 6 | 2 | 0.575 | 0.8 | 0.575 | 1.4 | 0.620 | 1.3 | 0.928 | 0.6 | 0.950 | 0.5 | 0.975 | 0.6 | 0.896 | 1.1 | 0.809 | 1.5 |
| 9 | 3 | 0.931 | 1.6 | 0.845 | 1.0 | 0.846 | 1.0 | 0.941 | 0.8 | 0.958 | 0.6 | 0.983 | 0.7 | 0.999 | 0.2 | 0.995 | 0.5 |

### EXAMPLE B9

Fenretinide and L-*threo*-sphinganines tested in human primitive neuroectodermal tumor (PNET) (brain cancer) cell line, CHLA-266. Results show that at least some L-*threo*-sphinganines increased fenretinide cytotoxicity, additively or synergistically, depending on the drug concentrations tested. Reference is made to the graphs plotting the results shown in **FIGS. 35A & 35B** which results are reported in **TABLE 9.1** below.

**TABLE 9.1**

| CHLA-266 | | | | | | | | | | | | | | | | | |
|---|---|---|---|---|---|---|---|---|---|---|---|---|---|---|---|---|---|
| 4-HPR | L-threo-Sphinganine | C12 | | C14 | | C15 | | C17 | | **C18** | | C19 | | C20 | | C21 | |
| (µM) | (µM) | Fₐ | Cl | Fₐ | Cl | Fₐ | Cl | Fₐ | Cl | Fₐ | Cl | Fₐ | Cl | Fₐ | Cl | Fₐ | Cl |
| 1.5 | 0.5 | 0.464 | 0.6 | | | | | 0.342 | 0.6 | 0.657 | 0.5 | 0.502 | 0.5 | 0.604 | 0.5 | | |
| 3 | 1 | 0.788 | 0.6 | | | | | 0.739 | 0.9 | 0.701 | 1.0 | 0.913 | 0.5 | 0.880 | 0.8 | | |
| 6 | 2 | 0.951 | 0.7 | | | | | 0.968 | 1.1 | 0.906 | 1.2 | 0.990 | 0.5 | 0.984 | 1.1 | | |
| 9 | 3 | 0.965 | 1.0 | | | | | 0.974 | 1.6 | 0.999 | 0.4 | 0.989 | 0.7 | 0.989 | 1.5 | | |

### EXAMPLE B10

Fenretinide and L-*threo*-sphinganines tested in human colorectal adenocarcinoma (colon cancer) cell line, HT-29. Results show that at least some L*-threo-*sphinganines increased fenretinide cytotoxicity, additively or synergistically, depending on the drug concentrations tested. Reference is made to the graphs plotting the results shown in **FIGS. 36A - 36D** which results are reported in **TABLE 10.1** below.

**TABLE 10.1**

| HT-29 | | | | | | | | | | | | | | | | | |
|---|---|---|---|---|---|---|---|---|---|---|---|---|---|---|---|---|---|
| 4-HPR | L-threo-Sphinganine | C12 | | C14 | | C15 | | C17 | | **C18** | | C19 | | C20 | | C21 | |
| (µM) | (µM) | Fₐ | Cl | Fₐ | Cl | Fₐ | Cl | Fₐ | Cl | Fₐ | Cl | Fₐ | Cl | Fₐ | Cl | Fₐ | Cl |
| 1.5 | 0.5 | 0.217 | 1.0 | 0.215 | 1.0 | 0.204 | 2.0 | 0.112 | 1.1 | 0.243 | 0.9 | 0.138 | 1.2 | 0.287 | 0.8 | 0.149 | 1.2 |
| 3 | 1 | 0.642 | 1.2 | 0.598 | 1.3 | 0.660 | 0.8 | 0.661 | 0.8 | 0.756 | 0.7 | 0.736 | 1.0 | 0.852 | 0.6 | 0.630 | 1.2 |
| 6 | 2 | 0.951 | 1.3 | 0.822 | 1.8 | 0.859 | 1.1 | 0.975 | 0.6 | 0.983 | 0.5 | 0.965 | 0.8 | 0.971 | 0.6 | 0.857 | 1.8 |
| 9 | 3 | 0.975 | 1.6 | 0.982 | 1.4 | 0.976 | 0.8 | 0.993 | 0.5 | 0.999 | 0.3 | 0.993 | 0.8 | 0.998 | 0.4 | 0.918 | 2.3 |

### EXAMPLE B11

Fenretinide and L-*threo*-sphinganines tested in human melanoma (skin cancer) cell line, A-2058. Results show that at least some L-*threo*-sphinganines increased fenretinide cytotoxicity, additively or synergistically, depending on the drug concentrations tested. Reference is made to the graphs plotting the results shown in **FIGS. 37A - 37D** which results are reported in **TABLE 11.1** below.

**TABLE 11.1**

| A-2058 | | | | | | | | | | | | | | | | | |
|---|---|---|---|---|---|---|---|---|---|---|---|---|---|---|---|---|---|
| 4-HPR | L-threo-Sphinganine | C12 | | C14 | | C15 | | C17 | | **C18** | | C19 | | C20 | | C21 | |
| (µM) | (µM) | Fₐ | Cl | Fₐ | Cl | Fₐ | Cl | Fₐ | Cl | Fₐ | Cl | Fₐ | Cl | Fₐ | Cl | Fₐ | Cl |
| 1.5 | 0.5 | 0.244 | 0.3 | 0.045 | 1.1 | 0.053 | 0.5 | 0.211 | 0.9 | 0.001 | >1 | 0.016 | 2.1 | 0.311 | 0.7 | 0.016 | 0.7 |
| 3 | 1 | 0.247 | 0.6 | 0.088 | 1.2 | 0.106 | 0.9 | 0.359 | 1.2 | 0.01 | >1 | 0.198 | 1.7 | 0.742 | 1.0 | 0.016 | 1.4 |
| 6 | 2 | 0.518 | 0.9 | 0.193 | 1.4 | 0.262 | 1.6 | 0.664 | 1.4 | 0.099 | >1 | 0.648 | 1.8 | 0.870 | 1.7 | 0.333 | 1.9 |
| 9 | 3 | 0.854 | 1.0 | 0.650 | 1.3 | 0.867 | 1.7 | 0.849 | 1.5 | 0.815 | 1.22 | 0.997 | 0.8 | 1.000 | 0.5 | 0.955 | 1.9 |

### EXAMPLE B12

Fenretinide and L-*threo*-sphinganines tested in human lung adenocarcinoma (lung cancer) cell line, NCI-H-1792. Results show that at least some L-*threo*-sphinganines increased fenretinide cytotoxicity, additively or synergistically, depending on the drug concentrations tested. Reference is made to the graphs plotting the results shown in **FIGS. 38A - 38B** which results are reported in **TABLE 12.1** below.

**TABLE 12.1**

| NCl-H-1792 | | | | | | | | | | | | | | | | | |
|---|---|---|---|---|---|---|---|---|---|---|---|---|---|---|---|---|---|
| 4-HPR | L-threo-Sphinganine | C12 | | C14 | | C15 | | C17 | | **C18** | | C19 | | C20 | | C21 | |
| (µM) | (µM) | Fₐ | Cl | Fₐ | Cl | Fₐ | Cl | Fₐ | Cl | Fₐ | Cl | Fₐ | Cl | Fₐ | Cl | Fₐ | Cl |
| 1.5 | 0.5 | 0.217 | 1.0 | | | | | 0.112 | 1.1 | 0.243 | 1.2 | 0.138 | 1.1 | 0.287 | 0.9 | | |
| 3 | 1 | 0.642 | 1.3 | | | | | 0.661 | 1.0 | 0.756 | 0.8 | 0.736 | 1.1 | 0.852 | 0.7 | | |
| 6 | 2 | 0.951 | 1.4 | | | | | 0.974 | 0.9 | 0.983 | 0.6 | 0.965 | 1.2 | 0.972 | 0.7 | | |
| 9 | 3 | 0.975 | 1.8 | | | | | 0.993 | 1.0 | 0.999 | 0.4 | 0.993 | 0.9 | 0.998 | 0.5 | | |

### EXAMPLE B13

Fenretinide and L-*threo*-sphinganines tested in human lung adenocarcinoma (lung cancer) cell line, A-549. Results show that at least some L-*threo*-sphinganines increased fenretinide cytotoxicity, additively or synergistically, depending on the drug concentrations tested. Reference is made to the graphs plotting the results shown in **FIGS. 39A - 39D** which results are reported in **TABLE 13.1** below.

**TABLE 13.1**

| A-549 | | | | | | | | | | | | | | | | | |
|---|---|---|---|---|---|---|---|---|---|---|---|---|---|---|---|---|---|
| 4-HPR | L-threo-Sphinganine | C12 | | C14 | | C15 | | C17 | | **C18** | | C19 | | C20 | | C21 | |
| (µM) | (µM) | Fₐ | Cl | Fₐ | Cl | Fₐ | Cl | Fₐ | Cl | Fₐ | Cl | Fₐ | Cl | Fₐ | Cl | Fₐ | Cl |
| 1.5 | 0.5 | 0.01 | 2.5 | 0.01 | 1 | 0.000 | 1.9 | 0.01 | 1.6 | 0.012 | 1.0 | 0.000 | 1.6 | 0.000 | 2.1 | 0.078 | 0.9 |
| 3 | 1 | 0.010 | 1.0 | 0.02 | 1.0 | 0.000 | 2.1 | 0.010 | 1.5 | 0.05 | 1.0 | 0.007 | 0.6 | 0.000 | 4.1 | 0.044 | 2.6 |
| 6 | 2 | 0.010 | 1.0 | 0.072 | 1.1 | 0.044 | 0.9 | 0.010 | 1.0 | 0.200 | 1.0 | 0.159 | 0.7 | 0.017 | 1.1 | 0.51 | 0.8 |
| 9 | 3 | 0.020 | 1.0 | 0.461 | 0.5 | 0.342 | 0.8 | 0.010 | 1.1 | 0.700 | 0.6 | 0.413 | 0.9 | 0.499 | 1.1 | 0.972 | 0.4 |

### EXAMPLE B14

Fenretinide and L-*threo*-sphinganines tested in human breast adenocarcinoma (breast cancer) cell lines, MCF-7 and MDA-MB-231. Results show that at least some L-*threo*-sphinganines increased fenretinide cytotoxicity, additively or synergistically, depending on the drug concentrations tested. Reference is made to the graphs plotting the results shown in **FIGS. 40A - 40D** which results are reported in **TABLES 14.1** and **14.2** below.

**TABLE 14.1**

| MCF-7 | | | | | | | | | | | | | | | | | |
|---|---|---|---|---|---|---|---|---|---|---|---|---|---|---|---|---|---|
| 4-HPR | L-threo-Sphinganine | C12 | | C14 | | C15 | | C17 | | **C18** | | C19 | | C20 | | C21 | |
| (µM) | (µM) | Fₐ | Cl | Fₐ | Cl | Fₐ | Cl | Fₐ | Cl | Fₐ | Cl | Fₐ | Cl | Fₐ | Cl | Fₐ | Cl |
| 1.5 | 0.5 | 0.217 | 1.0 | | | | | 0.112 | 1.3 | 0.243 | 1.2 | 0.138 | 1.2 | 0.287 | 0.8 | | |
| 3 | 1 | 0.642 | 1.0 | | | | | 0.661 | 0.7 | 0.756 | 0.6 | 0.736 | 0.8 | 0.852 | 0.4 | | |
| 6 | 2 | 0.951 | 0.9 | | | | | 0.974 | 0.4 | 0.824 | 0.9 | 0.965 | 0.8 | 0.971 | 0.3 | | |
| 9 | 3 | 0.975 | 1.2 | | | | | 0.993 | 0.4 | 0.961 | 0.6 | 0.991 | 0.9 | 0.998 | 0.1 | | |

**TABLE 14.2**

| MDA-MB-231, 20% O2 | | | | | | | | | | | | | | | |
|---|---|---|---|---|---|---|---|---|---|---|---|---|---|---|---|
| 4-HPR | L-threo-Sphinganine | C12 | | C14 | | C15 | | C17 | | **C18** | | C19 | | C20 | |
| (µM) | (µM) | Fₐ | Cl | Fₐ | Cl | Fₐ | Cl | Fₐ | Cl | Fₐ | Cl | Fₐ | Cl | Fₐ | Cl |
| 1.5 | 0.5 | 1E-07 | 1.5 | | | | | 0.127 | 1.3 | 0.088 | 1.8 | 0.078 | 1.2 | 0.011 | 2.0 |
| 3 | 1 | 0.052 | 0.9 | | | | | 0.265 | 0.9 | 0.207 | 1.8 | 0.195 | 1.2 | 0.190 | 1.1 |
| 6 | 2 | 0.272 | 1.6 | | | | | 0.610 | 0.8 | 0.597 | 1.3 | 0.503 | 1.1 | 0.583 | 1.3 |
| 9 | 3 | 0.720 | 2.0 | | | | | 0.948 | 0.7 | 0.922 | 0.7 | 0.909 | 0.7 | 0.664 | 1.8 |

### EXAMPLE B15

Fenretinide and L-*threo*-sphinganines tested in human ovarian adenocarcinoma (ovarian cancer) cell line, OVCAR-8. Results show that at least some L-*threo*-sphinganines increased fenretinide cytotoxicity, additively or synergistically, depending on the drug concentrations tested. Reference is made to the graphs plotting the results shown in **FIGS. 41A - 41D** which results are reported in **TABLE 15.1** below.

**TABLE 15.1**

| OVCAR-8 | | | | | | | | | | | | | | | | | |
|---|---|---|---|---|---|---|---|---|---|---|---|---|---|---|---|---|---|
| 4-HPR | L-threo-Sphinganine | C12 | | C14 | | C15 | | C17 | | **C18** | | C19 | | C20 | | C21 | |
| (µM) | (µM) | Fₐ | Cl | Fₐ | Cl | Fₐ | Cl | Fₐ | Cl | Fₐ | Cl | Fₐ | Cl | Fₐ | Cl | Fₐ | Cl |
| 1.5 | 0.5 | 0.382 | 0.3 | 0.696 | 0.1 | 0.668 | 0.1 | 0.467 | 0.2 | 0.122 | 1.5 | 0.787 | 0.1 | 0.259 | 0.5 | 0.626 | 0.1 |
| 3 | 1 | 0.445 | 0.5 | 0.860 | 0.1 | 0.854 | 0.1 | 0.519 | 0.4 | 0.202 | 1.5 | 0.881 | 0.1 | 0.366 | 0.6 | 0.870 | 0.1 |
| 6 | 2 | 0.634 | 0.6 | 0.978 | 0.1 | 0.977 | 0.1 | 0.676 | 0.5 | 0.536 | 0.7 | 0.943 | 0.1 | 0.453 | 0.8 | 0.977 | 0.1 |
| 9 | 3 | 0.704 | 0.8 | 0.988 | 0.1 | 0.977 | 0.2 | 0.903 | 0.4 | 0.761 | 0.5 | 0.978 | 0.1 | 0.956 | 0.2 | 0.975 | 0.2 |

### EXAMPLE B16

Fenretinide and L-*threo*-sphinganines tested in human prostate adenocarcinoma (prostate cancer) cell line, LNCaP. Results show that at least some L-*threo-*sphinganines increased fenretinide cytotoxicity, additively or synergistically, depending on the drug concentrations tested. Reference is made to the graphs plotting the results shown in **FIGS. 42A - 42B** which results are reported in **TABLE 16.1** below.

**TABLE 16.1**

| LNCaP.FGC | | | | | | | | | | | | | | | |
|---|---|---|---|---|---|---|---|---|---|---|---|---|---|---|---|
| 4-HPR | L-threo-Sphinganine | C12 | | C14 | | C15 | | C17 | | **C18** | | C19 | | C20 | |
| (µM) | (µM) | Fₐ | Cl | Fₐ | Cl | Fₐ | Cl | Fₐ | Cl | Fₐ | Cl | Fₐ | Cl | Fₐ | Cl |
| 1.5 | 0.5 | 0.870 | 0.5 | | | | | 0.887 | 0.3 | 0.752 | 0.9 | 0.697 | 1.0 | 0.812 | 0.7 |
| 3 | 1 | 0.982 | 0.3 | | | | | 0.967 | 0.3 | 0.971 | 0.3 | 0.980 | 0.5 | 0.987 | 0.4 |
| 6 | 2 | 0.996 | 0.4 | | | | | 0.991 | 0.2 | 0.998 | 0.1 | 0.999 | 0.5 | 0.993 | 0.7 |
| 9 | 3 | 0.999 | 0.4 | | | | | 0.999 | 0.1 | 0.999 | 0.1 | 1.000 | 0.2 | 0.999 | 0.8 |

### EXAMPLE B17

Fenretinide and L-*threo*-sphinganines tested in human prostate adenocarcinoma (prostate cancer) cell line, PC-3. Results show that at least some L*-threo-*sphinganines increased fenretinide cytotoxicity, additively or synergistically, depending on the drug concentrations tested. Reference is made to the graphs plotting the results shown in **FIGS. 43A - 43D** which results are reported in **TABLE 17.1** below.

**TABLE 17.1**

| PC-3 | | | | | | | | | | | | | | | | | |
|---|---|---|---|---|---|---|---|---|---|---|---|---|---|---|---|---|---|
| 4-HPR | L-threo-Sphinganine | C12 | | C14 | | C15 | | C17 | | **C18** | | C19 | | C20 | | C21 | |
| (µM) | (µM) | Fₐ | Cl | F**ₐ** | Cl | Fₐ | Cl | Fₐ | Cl | Fₐ | Cl | Fₐ | Cl | Fₐ | Cl | F**ₐ** | Cl |
| 1.5 | 0.5 | 0.245 | 0.5 | 0.192 | 0.7 | 0.200 | 0.6 | 0.214 | 0.7 | 0.202 | 1.1 | 0.233 | 1.0 | 0.217 | 1.0 | 0.215 | 1.7 |
| 3 | 1 | 0.328 | 0.8 | 0.283 | 1.0 | 0.287 | 0.9 | 0.338 | 0.9 | 0.346 | 1.4 | 0.406 | 1.1 | 0.393 | 1.1 | 0.350 | 1.4 |
| 6 | 2 | 0.526 | 0.9 | 0.426 | 1.3 | 0.417 | 1.2 | 0.623 | 0.8 | 0.719! | 1.1 | 0.635 | 1.1 | 0.620 | 1.2 | 0.479 | 1.6 |
| 9 | 3 | 0.721 | 0.8 | 0.634 | 1.2 | 0.621 | 1.0 | 0.862 | 0.5 | 0.918 | 0.7 | 0.913 | 0.5 | 0.916 | 0.5 | 0.738 | 0.9 |

### EXAMPLE B18

Fenretinide and L-*threo*-sphinganines tested in human pancreatic adenocarcinoma (pancreas cancer) cell line, PANC-1. Results show that at least some L-*threo*-sphinganines increased fenretinide cytotoxicity, additively or synergistically, depending on the drug concentrations tested. Reference is made to the graphs plotting the results shown in **FIGS. 44A - 44D** which results are reported in **TABLE 18.1** below.

**TABLE 18.1**

| PANC-1 | | | | | | | | | | | | | | | | | |
|---|---|---|---|---|---|---|---|---|---|---|---|---|---|---|---|---|---|
| 4-HPR | L-threo-Sphinganine | C12 | | C14 | | C15 | | C17 | | **C18** | | C19 | | C20 | | C21 | |
| (µM) | (µM) | Fₐ | Cl | Fₐ | Cl | Fₐ | Cl | Fₐ | Cl | Fₐ | Cl | Fₐ | Cl | Fₐ | Cl | Fₐ | Cl |
| 1.5 | 0.5 | 0.232 | 0.9 | 0.054 | 3.2 | 0.078 | 2.9 | 0.171 | 1.5 | 0.136 | 2.5 | 0.182 | 1.5 | 0.136 | 1.9 | 0.111 | 2.5 |
| 3 | 1 | 0.119 | 3.1 | 0.176 | 2.6 | 0.205 | 2.2 | 0.311 | 1.7 | 0.336 | 2.1 | 0.321 | 1.8 | 0.291 | 1.9 | 0.239 | 2.9 |
| 6 | 2 | 0.757 | 0.7 | 0.450 | 2.1 | 0.486 | 1.7 | 0.714 | 0.9 | 0.620 | 1.4 | 0.663 | 1.4 | 0.653 | 1.2 | 0.784 | 1.6 |
| 9 | 3 | 0.977 | 0.2 | 0.883 | 0.7 | 0.978 | 0.2 | 0.990 | 0.1 | 0.700 | 1.5 | 0.997 | 0.1 | 0.990 | 0.1 | 0.994 | 0.4 |

### EXAMPLE B19

Fenretinide and L-*threo*-sphinganines tested in human esophageal adenocarcinoma (esophagus cancer) cell lines, OE-19 and OE-33. Results show that at least some L-*threo*-sphinganines increased fenretinide cytotoxicity, additively or synergistically, depending on the drug concentrations tested. Reference is made to the graphs plotting the results shown in **FIGS. 45A - 45D** which results are reported in **TABLES 19.1** and **19.2** below.

**TABLE 19.1**

| OE-19 | | | | | | | | | | | | | | | |
|---|---|---|---|---|---|---|---|---|---|---|---|---|---|---|---|
| 4-HPR | L-threo-Sphinganine | C12 | | C14 | | C15 | | C17 | | **C18** | | C19 | | C20 | |
| (µM) | (µM) | Fₐ | Cl | Fₐ | Cl | F**ₐ** | Cl | Fₐ | Cl | Fₐ | Cl | Fₐ | Cl | Fₐ | Cl |
| 1.5 | 0.5 | 0.345 | 1.4 | | | | | 0.314 | 0.3 | 0.281 | 0.7 | 0.094 | 1.0 | 0.352 | 3.2 |
| 3 | 1 | 0.325 | 2.9 | | | | | 0.462 | 0.3 | 0.464 | 0.4 | 0.109 | 1.6 | 0.438 | 6.6 |
| 6 | 2 | 0.506 | 5.5 | | | | | 0.689 | 0.2 | 0.709 | 0.2 | 0.173 | 1.6 | 0.708 | >>1 |
| 9 | 3 | 0.632 | >>1 | | | | | 0.743 | 0.2 | 0.799 | 0.2 | 0.272 | 1.1 | 0.771 | >>1 |

**TABLE 19.2**

| OE-33 | | | | | | | | | | | | | | | | | |
|---|---|---|---|---|---|---|---|---|---|---|---|---|---|---|---|---|---|
| 4-HPR | L-threo-Sphinganine | C12 | | C14 | | C15 | | C17 | | C18 | | C19 | | C20 | | C21 | |
| (µM) | (µM) | Fₐ | Cl | Fₐ | Cl | Fₐ | Cl | Fₐ | Cl | Fₐ | Cl | Fₐ | Cl | Fₐ | Cl | Fₐ | Cl |
| 1.5 | 0.5 | 0.376 | 1.0 | | | | | 0.376 | 1.5 | | | 0.299 | 1.9 | 0.255 | 1.9 | | |
| 3 | 1 | 0.618 | 1.0 | | | | | 0.767 | 1.1 | | | 0.582 | 1.6 | 0.445 | 1.9 | | |
| 6 | 2 | 0.758 | 1.3 | | | | | 0.945 | 0.8 | | | 0.951 | 0.6 | 0.883 | 0.8 | | |
| 9 | 3 | 0.925 | 1.1 | | | | | 0.991 | 0.4 | | | 0.989 | 0.3 | 0.993 | 0.2 | | |

### EXAMPLE B20

Fenretinide and L-*threo*-sphinganines tested in human acute lymphoblastic leukemia (pediatric ALL, a blood cancer) cell lines, COG-LL-317 and MOLT-4. Results show that at least some L-*threo*-sphinganines increased fenretinide cytotoxicity, additively or synergistically, depending on the drug concentrations tested. Reference is made to the graphs plotting the results shown in **FIGS. 46A - 46D** which results are reported in **TABLES 20.1** and **20.2** below.

**TABLE 20.1**

| COG-LL-317 | | | | | | | | | | | | | | | |
|---|---|---|---|---|---|---|---|---|---|---|---|---|---|---|---|
| 4-HPR | L-threo-Sphinganine | C12 | | C14 | | C15 | | C17 | | **C18** | | C19 | | C20 | |
| (µM) | (µM) | Fₐ | Cl | Fₐ | Cl | Fₐ | Cl | Fₐ | Cl | Fₐ | Cl | Fₐ | Cl | Fₐ | Cl |
| 3 | 1 | | | | | | | 0.99300 | 0.8 | 0.99800 | 0.8 | 0.9980 | 0.8 | 0.99990 | 0.2 |
| 6 | 2 | | | | | | | 0.99930 | 1.3 | 0.99940 | 0.6 | 0.9999 | 0.4 | 1.00000 | 0.3 |
| 9 | 3 | | | | | | | 0.99993 | 0.5 | 0.99960 | 0.8 | 1.0000 | na | 1.00000 | na |
| 12 | 4 | | | | | | | 1.00000 | na | 1.00000 | na | 1.0000 | na | 1.00000 | na |

**TABLE 20.2**

| MOLT-4 | | | | | | | | | | | | | | | |
|---|---|---|---|---|---|---|---|---|---|---|---|---|---|---|---|
| 4-HPR | L-threo-Sphinganine | C12 | | C14 | | C15 | | C17 | | **C18** | | C19 | | C20 | |
| (µM) | (µM) | Fₐ | Cl | Fₐ | Cl | Fₐ | Cl | Fₐ | Cl | Fₐ | Cl | Fₐ | Cl | Fₐ | Cl |
| 3 | 1 | | | | | | | 0.807 | 1.0 | 0.829 | 0.9 | 0.797 | 1.0 | 0.878 | 0.9 |
| 6 | 2 | | | | | | | 0.998 | 0.7 | 0.998 | 0.7 | 0.996 | 0.8 | 0.996 | 0.8 |
| 9 | 3 | | | | | | | 0.999 | 0.8 | 0.999 | 0.8 | 0.999 | 0.8 | 0.999 | 0.8 |
| 12 | 4 | | | | | | | 1 | na | 1 | na | 1 | na | 1 | na |

### EXAMPLE B21

Fenretinide and L-*threo*-sphinganines tested in human pediatric neuroblastoma (a nerve-related, solid tumor cancer) cell line, CHLA-90. Results show that at least some L-*threo*-sphinganines increased fenretinide cytotoxicity, additively or synergistically, depending on the drug concentrations tested. Reference is made to the graphs plotting the results shown in **FIGS. 47A** - **47B** which results are reported in **TABLE 21.1** below.

**TABLE 21.1**

| CHLA-90 | | | | | | | | | | | | | | | |
|---|---|---|---|---|---|---|---|---|---|---|---|---|---|---|---|
| 4-HPR | L-threo-Sphinganine | C12 | | C14 | | C15 | | C17 | | **C18** | | C19 | | C20 | |
| (µM) | (µM) | Fₐ | Cl | Fₐ | Cl | Fₐ | Cl | Fₐ | Cl | **Fₐ** | **Cl** | Fₐ | Cl | Fₐ | Cl |
| 3 | 1 | | | | | | | 0.99400 | 0.4 | 0.99600 | 0.5 | 0.9960 | 0.4 | 0.996 | 0.5 |
| 6 | 2 | | | | | | | 0.99900 | 0.4 | 0.99930 | 0.3 | 0.9993 | 0.1 | 0.999 | 0.7 |
| 9 | 3 | | | | | | | 0.99970 | 0.6 | 0.99992 | 0.4 | 0.99995 | 0.1 | 0.999 | 1.1 |
| 12 | 4 | | | | | | | 0.99996 | 0.3 | 1.00000 | na | 1.0000 | na | 0.9999 | 1.5 |

The foregoing is illustrative of the present invention, and is not to be construed as limiting thereof. The invention is defined by the following claims.

To facilitate the understanding of this invention, a number of terms are defined below. Terms defined herein have meanings as commonly understood by a person of ordinary skill in the areas relevant to the present invention. Terms such as "a", "an", and "the" are not intended to refer to only a singular entity, but include the general class of which a specific example may be used for illustration.

In the claims, all transitional phrases such as "comprising," "carrying," "having," "containing" and "involving" are to be understood to be openended, i.e., to mean including . Only the transitional phrases "consisting of" and "consisting essentially of," respectively, shall be closed or semi-closed transitional phrases.

All of the compositions and methods disclosed and claimed herein can be made and executed without undue experimentation in light of the present disclosure.

## Claims

1. A non-18 carbon chain length L-threo-sphinganine having the formula I: 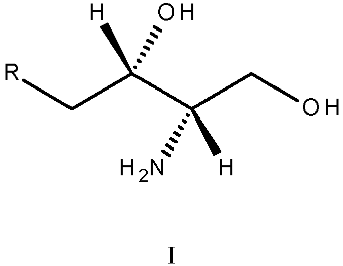
wherein R is a linear saturated hydrocarbon chain, and is such that the overall carbon chain length is 15 carbons, 17 carbons, 19 carbons, 20 carbons, or 21 carbons,
for use in therapy.

2. A pharmaceutical L-threo-sphinganine composition comprising:
a ceramide-increasing retinoid and
a non-18 carbon chain length L-threo-sphinganine derivative having the formula I 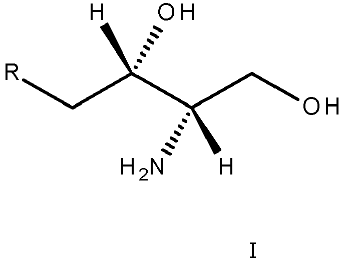
in a pharmaceutical carrier, wherein R is a linear saturated hydrocarbon chain, and is such that the overall carbon chain length is 15 carbons, 17 carbons, 19 carbons, 20 carbons, or 21 carbons.

3. A medicament for the use in a treatment of a hyperproliferative disorder in a subject, wherein the medicament comprises:
a ceramide-increasing retinoid and
a non-18 carbon chain length L-threo-sphinganine having Formula I: 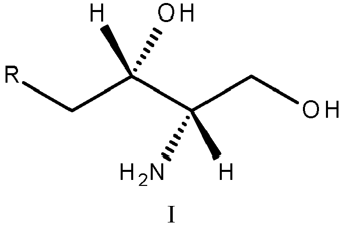
wherein R is a linear saturated hydrocarbon chain, and is such that the overall carbon chain length is 15 carbons, 17 carbons, 19 carbons, 20 carbons, or 21 carbons,
in a pharmaceutical carrier.

4. The compound for use according to claim 1, further comprising a ceramide-increasing retinoid.

5. The compound for use or the composition according to any of claims 1-3, wherein the non-18 carbon chain length L-threo-sphinganine has a carbon chain length of 17 carbons, 19 carbons, or 20 carbons.

6. The compound for use or the composition according to claim 5, wherein the non-18 carbon chain length L-threo-sphinganine is L-threo-C20-sphinganine.

7. The compound for use or the composition according to claim 5, wherein the non-18 carbon chain length L-threo-sphinganine is L-threo-C17-sphinganine.

8. The compound for use or the composition according to any of claims 2-4, wherein the ceramide-increasing retinoid is fenretinide.

9. The compound for use or the composition according to any of claims 2-4, further comprising a glucosylceramide or glucosyl(dihydro)ceramide synthase inhibitor.

10. The compound for use or the composition according to claim 9, wherein the glucosylceramide or glucosyl(dihydro)ceramide synthase inhibitor is 1-phenyl-2-palmitoylamino-3-morpholino-1-propanol.

11. The compound for use or the composition according to claim 9, wherein the glucosylceramide or glucosyl(dihydro)ceramide synthase inhibitor is D-threo-1-phenyl-2-palmitoylamino-3-morpholino-1-propanol.

12. The composition or medicament for use according to of any of claims 2 or 3, further comprising a sphingomyelin or (dihydro)sphingomyelin synthase inhibitor.

13. The composition or medicament for use according to claim 12, wherein the sphingomyelin or (dihydro)sphingomyelin synthase inhibitor(s) is D-threo-1-phenyl-2-palmitoylamino-3- morpholino-1-propanol.

14. The medicament for use according to claim 3, wherein the hyperproliferative disorder is a cancer.

## Patentansprüche

1. L-threo-Sphinganin mit einer Kettenlänge, die nicht 18 Kohlenstoffe beträgt, mit der Formel I: 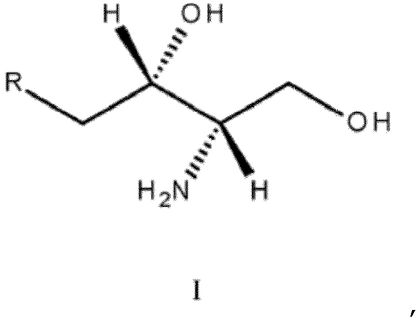
wobei R für eine geradkettige gesättigte Kohlenwasserstoffkette steht und so beschaffen ist, dass die Gesamtkohlenstoffkettenlänge 15 Kohlenstoffe, 17 Kohlenstoffe, 19 Kohlenstoffe, 20 Kohlenstoffe oder 21 Kohlenstoffe beträgt,
zur Verwendung in der Therapie.

2. Pharmazeutische L-threo-Sphinganin-Zusammensetzung, umfassend:
ein ceramiderhöhendes Retinoid und
ein L-threo-Sphinganin-Derivat mit einer Kettenlänge, die nicht 18 Kohlenstoffe beträgt, mit der Formel I 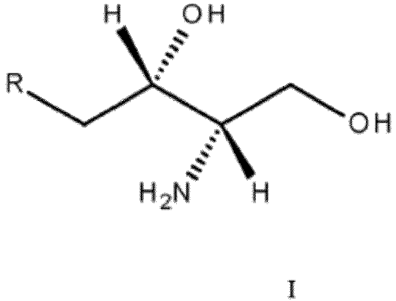 in einem pharmazeutischen Träger, wobei R für eine geradkettige gesättigte Kohlenwasserstoffkette steht und so beschaffen ist, dass die Gesamtkohlenstoffkettenlänge 15 Kohlenstoffe, 17 Kohlenstoffe, 19 Kohlenstoffe, 20 Kohlenstoffe oder 21 Kohlenstoffe beträgt.

3. Medikament zur Verwendung bei der Behandlung einer hyperproliferativen Erkrankung in einem Subjekt, wobei das Medikament Folgendes umfasst:
ein ceramiderhöhendes Retinoid und
ein L-threo-Sphinganin mit einer Kettenlänge, die nicht 18 Kohlenstoffe beträgt, mit der Formel I: 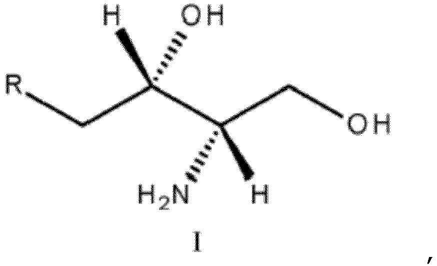
wobei R für eine geradkettige gesättigte Kohlenwasserstoffkette steht und so beschaffen ist, dass die Gesamtkohlenstoffkettenlänge 15 Kohlenstoffe, 17 Kohlenstoffe, 19 Kohlenstoffe, 20 Kohlenstoffe oder 21 Kohlenstoffe beträgt,
in einem pharmazeutischen Träger.

4. Verbindung zur Verwendung nach Anspruch 1, welche weiterhin ein ceramiderhöhendes Retinoid umfasst.

5. Verbindung zur Verwendung oder Zusammensetzung nach einem der Ansprüche 1-3, wobei das L-threo-Sphinganin mit einer Kettenlänge, die nicht 18 Kohlenstoffe beträgt, eine Kettenlänge von 17 Kohlenstoffen, 19 Kohlenstoffen oder 20 Kohlenstoffen aufweist.

6. Verbindung zur Verwendung oder Zusammensetzung nach Anspruch 5, wobei das L-threo-Sphinganin mit einer Kettenlänge, die nicht 18 Kohlenstoffe beträgt, L-threo-C20-Sphinganin ist.

7. Verbindung zur Verwendung oder Zusammensetzung nach Anspruch 5, wobei das L-threo-Sphinganin mit einer Kettenlänge, die nicht 18 Kohlenstoffe beträgt, L-threo-C17-Sphinganin ist.

8. Verbindung zur Verwendung oder Zusammensetzung nach einem der Ansprüche 2-4, wobei es sich bei dem ceramiderhöhenden Retinoid um Fenretinid handelt.

9. Verbindung zur Verwendung oder Zusammensetzung nach einem der Ansprüche 2-4, weiterhin umfassend einen Glucosylceramid- oder Glucosyl(dihydro)ceramid-Synthase-Inhibitor.

10. Verbindung zur Verwendung oder Zusammensetzung nach Anspruch 9, wobei es sich bei dem Glucosylceramid- oder Glucosyl(dihydro)ceramid-Synthase-Inhibitor um 1-Phenyl-2-palmitoylamino-3-morpholino-1-propanol handelt.

11. Verfahren zur Verwendung oder Zusammensetzung nach Anspruch 9, wobei es sich bei dem Glucosylceramid- oder Glucosyl(dihydro)ceramid-Synthase-Inhibitor um D-threo-1-Phenyl-2-palmitoylamino-3-morpholino-1-propanol handelt.

12. Zusammensetzung oder Medikament zur Verwendung nach einem der Ansprüche 2 oder 3, weiterhin umfassend einen Sphingomyelin- oder (Dihydro)sphingomyelin-Synthase-Inhibitor.

13. Zusammensetzung oder Medikament zur Verwendung nach Anspruch 12, wobei es sich bei dem Sphingomyelin- oder (Dihydro)sphingomyelin-Synthase-Inhibitor um D-threo-1-Phenyl-2-palmitoylamino-3-morpholino-1-propanol handelt.

14. Medikament zur Verwendung nach Anspruch 3, wobei es sich bei der hyperproliferativen Erkrankung um Krebs handelt.

## Revendications

1. L-Thréo-sphinganine de longueur de chaîne carbonée différente de 18 répondant à la formule I : 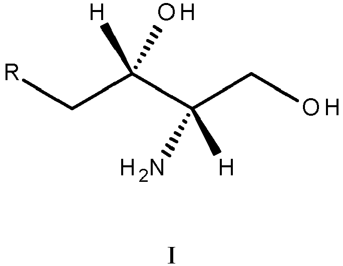
où R représente une chaîne hydrocarbonée saturée linéaire, et est tel que la longueur totale de la chaîne carbonée soit de 15 atomes de carbone, 17 atomes de carbone, 19 atomes de carbone, 20 atomes de carbone ou 21 atomes de carbone,
pour utilisation en thérapie.

2. Composition de L-thréo-sphinganine pharmaceutique comprenant :
un rétinoïde augmentant les céramides et
un dérivé de L-thréo-sphinganine de longueur de chaîne carbonée différente de 18 répondant à la formule I : 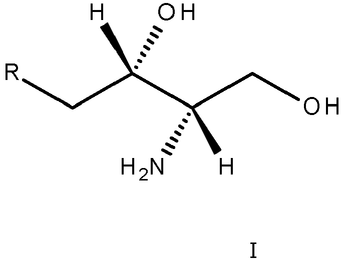 dans un vecteur pharmaceutique, où R représente une chaîne hydrocarbonée saturée linéaire, et est tel que la longueur totale de la chaîne carbonée soit de 15 atomes de carbone, 17 atomes de carbone, 19 atomes de carbone, 20 atomes de carbone ou 21 atomes de carbone.

3. Médicament pour utilisation dans le traitement d'un trouble hyperprolifératif chez un sujet, où le médicament comprend :
un rétinoïde augmentant les céramides et
une L-thréo-sphinganine de longueur de chaîne carbonée différente de 18 répondant à la Formule I : 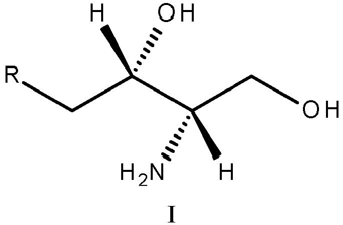
où R représente une chaîne hydrocarbonée saturée linéaire, et est tel que la longueur totale de la chaîne carbonée soit de 15 atomes de carbone, 17 atomes de carbone, 19 atomes de carbone, 20 atomes de carbone ou 21 atomes de carbone,
dans un vecteur pharmaceutique.

4. Composé pour utilisation selon la revendication 1, comprenant en outre un rétinoïde augmentant les céramides.

5. Composé pour utilisation ou composition selon l'une quelconque des revendications 1 à 3, où la L-thréo-sphinganine de longueur de chaîne carbonée différente de 18 présente une longueur de chaîne carbonée de 17 atomes de carbone, 19 atomes de carbone ou 20 atomes de carbone.

6. Composé pour utilisation ou composition selon la revendication 5, où la L-thréo-sphinganine de longueur de chaîne carbonée différente de 18 est la L-thréo-C20-sphinganine.

7. Composé pour utilisation ou composition selon la revendication 5, où la L-thréo-sphinganine de longueur de chaîne carbonée différente de 18 est la L-thréo-C17-sphinganine.

8. Composé pour utilisation ou composition selon l'une quelconque des revendications 2 à 4, où le rétinoïde augmentant les céramides est le fenrétinide.

9. Composé pour utilisation ou composition selon l'une quelconque des revendications 2 à 4, comprenant en outre un inhibiteur de glucosylcéramide ou glucosyl(dihydro)céramide synthase.

10. Composé pour utilisation ou composition selon la revendication 9, où l'inhibiteur de glucosylcéramide ou glucosyl(dihydro)céramide synthase est le 1-phényl-2-palmitoylamino-3-morpholino-1-propanol.

11. Composé pour utilisation ou composition selon la revendication 9, où l'inhibiteur de glucosylcéramide ou glucosyl(dihydro)céramide synthase est le D-thréo-1-phényl-2-palmitoylamino-3-morpholino-1-propanol.

12. Composition ou médicament pour utilisation selon l'une quelconque des revendications 2 ou 3, comprenant en outre un inhibiteur de sphingomyéline ou (dihydro)sphingomyéline synthase.

13. Composition ou médicament pour utilisation selon la revendication 12, où l'inhibiteur de sphingomyéline ou (dihydro)sphingomyéline synthase est le D-thréo-1-phényl-2-palmitoylamino-3-morpholino-1-propanol.

14. Médicament pour utilisation selon la revendication 3, où le trouble hyperprolifératif est un cancer.
